# EUROPEAN PATENT APPLICATION

(11) **EP 3 693 011 A1**
(43) Date of publication of application: **12.08.2020**
(21) Application number: 20153001.1
(22) Date of filing: 31.03.2015
(51) Int. Cl.: A61K 39/39, A61K 47/34, A61K 47/36, A61K 9/16, A61P 31/14, A61P 37/04, A61P 31/04, A61K 47/10, A61K 31/47, A61K 9/00, A61K 39/12

(54) **PARTICULATE VACCINE FORMULATIONS FOR INDUCING INNATE AND ADAPTIVE IMMUNITY**

(30) Priority: 18.04.2014 US 201461981328 P; 30.04.2014 US 201461986148 P
(62) Divisional of application: 15719034.9
(71) Applicant: Auburn University, Auburn, AL 36832 (US)
(72) Inventor: KALTENBOECK, Bernhard, Auburn, AL 36830 (US); GUPTA, Ram B., Auburn, AL 36830 (US); CHOWDHURY, Erfan U., Auburn, AL 36830 (US); OBER, Courtney A., Carlisle, PA 17013 (US)
(74) Representative: Boult Wade Tennant LLP

(57) **Abstract**

Disclosed are compositions, kits, and methods for inducing an immune response against an infection or a disease. The compositions typically include biodegradable particles having an average effective diameter of 0.5 - 20 µm, and optionally the compositions include one or more of an adjuvant, an apoptosis inhibitor, and an antigen. The compositions, kits, and methods may be utilized to induce a cell-mediated response, such as a T-helper cell response, and/or a humoral response against a pathogen or a disease. In some embodiments, the compositions, kits, and methods may be utilized to induce preferentially a Th1 response versus other types of immune responses such as a Th2 response.

## Description

### CROSS-REFERENCE TO RELATED APPLICATIONS

This application claims the benefit under 35 U.S.C. § 119(e) to U.S. Provisional Application No. 61/981,328, filed on April 18, 2014 and to U.S. Provisional Application No. 61/986,148, filed on April 30, 2014, the contents of which is incorporated herein by reference in their entireties.

### BACKGROUND

The present invention relates generally to the field of compositions, kits, and methods for inducing an immune response. In particular, the invention relates to particulate vaccine formulations for inducing innate or adaptive immunity against an infection or a disease.

T-helper (Th) lymphocytes may be categorized into two distinct subsets of effector cells based on their functional capabilities and cytokine profiles. Th1 cells produce IFN-γ, TNF-β, and IL-2 and help to activate macrophages and cytotoxic T lymphocytes. In addition, Th1 cells assist other immune cells in the production of those antibody isotypes that promote opsonization. Th2 cells trigger B cells to produce and secrete antibodies. In contrast, Th2 cells are particularly effective at inducing B cells to produce certain antibody isotypes such as IgE and IgA, which neutralize intercellular pathogens and help opsonization, complement, mast cell, and eosinophil activation. Because of these functional differences, Th1 and Th2 exhibit different efficiency in elimination of a selected pathogen. Diseases that can be prevented or treated successfully by Th1 responses include mycobacterial infections such as tuberculosis, leprosy, leishmaniasis, and schistosomiasis, which are intracellular infections, and certain viral diseases. Th2 responses are protective against helminths and some bacteria such as pneumo- and meningococcii.

Th1 and Th2 cells arise from a common precursor cell called Th0. Differentiation of T-helper cells into Th1 and Th2 cells is an important event in determining the outcome of an immune response (*i.e.,* whether a pathogen will persist, whether the host will be protected, and/or whether the host will experience immunopathogenesis). Infectious pathogens may exhibit a predisposition to induce a cell-mediated form of immunity versus a humoral form of immunity. Successful defense against intracellular pathogens tends to be associated with Th1 dominance and resultant cellular cytolytic activity, whereas resistance to extracellular infectious pathogens is most often dominated by Th2 effectors, which lead to the production of high levels of antigen-specific immunoglobulins. Therefore, a better understanding of the factors that contribute to differentiation of Th0 cells into Th1 and Th2 cells will help facilitate preparation of more effective prevention and treatment strategies.

### SUMMARY

Disclosed are compositions, kits, and methods for inducing an immune response. The immune response induced by the composition, kits, and methods preferably is a Th1 cell immune response versus a Th2 cell immune response.

The compositions and kits disclosed herein include biodegradable particles having an effective average diameter of 0.5-10 um that have been shown herein to be effective in stimulating an innate or adaptive immune response. As such, particulate immunogenic compositions and vaccine formulations for inducing an innate or adaptive immune response are disclosed herein.

The disclosed particles of the compositions and formulations are biodegradable and may include polymeric or non-polymeric material. In some embodiments, the biodegradable particles comprise polymeric material formed from carbohydrate monomers. The biodegradable particles may be formed by a process that includes spray-drying a liquid composition to form the biodegradable particles.

The compositions and formulations optionally may include excipients for the biodegradable particles. In some embodiments, the compositions and formulations include a powder excipient. In other embodiments, the compositions and formulations comprise a suspension of the biodegradable particles in an excipient that includes a non-ionic surfactant solution.

The disclosed compositions and formulations comprising the biodegradable particles may be administered to a subject in order to induce an immune response. In some embodiments, the disclosed compositions and formulations are administered to the subject at a dose that delivers the biodegradable particles to the subject in an amount between about (BW/20)^{3/4} µg and 100 x ((BW/20)^{3/4}) µg, wherein BW is the body weight of the subject in grams.

The disclosed compositions and formulations comprising the biodegradable particles may include additional agents for modulating an immune response. In some embodiments, the disclosed compositions and formulations comprising the biodegradable particles further comprise an adjuvant. In even further embodiments, the disclosed compositions and formulations comprising the biodegradable particles further comprise an apoptosis inhibitor.

In some embodiments, the disclosed compositions and formulations comprising the biodegradable particles may be administered to a subject in a method for inducing innate immunity in the subject. For example, the compositions and formulations may consist of the biodegradable particles and optionally an adjuvant and/or an apoptosis inhibitor, and the vaccine formulation may not comprise an antigen for inducing adaptive immunity.

In other embodiments, the disclosed compositions and formulations comprising the biodegradable particles may be administered to a subject in a method for inducing adaptive immunity. For example, the compositions and formulations may comprise the biodegradable particles and optionally an adjuvant and/or an apoptosis inhibitor, and the compositions and formulations further may comprise an antigen for inducing adaptive immunity.

In embodiments in which the compositions and formulations comprising the biodegradable particles further comprise an antigen for inducing adaptive immunity, the antigen may be present at a concentration that is relative to the concentration of the biodegradable particles. In some embodiments, the compositions and formulations comprise particles and antigens at a molar ratio of 0.2, 0.5, 1.0, 2.0, or 5.0, and preferably at a molar ratio approaching 1.0. In embodiments in which the antigens are small peptide antigens (*e.g*., peptide antigens having 10-50 amino acids), the peptide antigen may present in the compositions and formulations at a suitable concentration ratio such as 0.00018 antigen/µg biodegradable particles, 0.0018 fmole antigen/µg biodegradable particles, 0.018 fmole antigen/µg biodegradable particles, 0.18 fmole antigen/µg biodegradable particles, 1.8 fmole antigen/µg biodegradable particles, 18.0 fmole antigen/µg biodegradable particles, and ratios within ranges defined by any pairs of these suitable ratios (*e.g*., 0.18 - 1.8 fmole antigen/µg biodegradable particles).

In embodiments in which the compositions and formulations comprising the biodegradable particles further comprise an antigen for inducing adaptive immunity, the vaccine formulations may be administered to the subject at a dose that delivers the antigen to the subject at a suitable dose level. In some embodiments, the compositions and formulations may be administered to the subject at a suitable dose levels such as 0.0009 fmole antigen/ g body weight of the subject, 0.009 fmole antigen/g body weight of the subject, 0.09 fmole antigen/ g body weight of the subject, 0.9 fmole antigen/ g body weight of the subject, and dose levels within ranges defined by any pairs of these suitable dose levels (*e.g.,* 0.09-0.9 fmole antigen/ g body weight of the subject). In other embodiments, the compositions and formulations may be administered to the subject at a suitable dose level such as 0.002 pg antigen/g body weight of the subject, 0.02 pg antigen/g body weight of the subject, 0.2 pg antigen/g body weight of the subject, 2.0 pg antigen/g body weight of the subject and dose levels within ranges defined by any pairs of these suitable dose levels (*e.g.,* 0.2-2.0 pg antigen/ g body weight of the subject).

Suitable antigens for the compositions and formulations comprising the biodegradable particles may include peptide antigens. For example, suitable antigens may include peptide antigens having an amino acid length of less than about 100, 50, 40, 30, or 20 amino acids. Suitable antigens may include peptide antigens having a molecular weight of less than about 10, 5, 4, 3, or 2kD.

The methods contemplated herein include methods that consist of administering compositions and formulations consisting essentially of the biodegradable particles. In some embodiments, the methods consist of administering compositions and formulations consisting essentially of a suspension of the biodegradable particles, such as a suspension of the biodegradable particles in a solution of a non-ionic surfactant. In other embodiments, the methods consist of administering compositions and formulations consisting essentially of a suspension of the biodegradable particles, such as a suspension of the biodegradable particles in a solution of a non-ionic surfactant and an adjuvant. In even further embodiments, the methods consist of administering compositions and formulations consisting essentially of a suspension of the biodegradable particles, such as a suspension of the biodegradable particles in a solution of a non-ionic surfactant, an adjuvant, and an apoptosis inhibitor. In even further embodiments, the methods consist of administering compositions and formulations consisting essentially of a suspension of the biodegradable particles, such as a suspension of the biodegradable particles in a solution of a non-ionic surfactant, an adjuvant, an apoptosis inhibitor, and an antigen (*e.g.,* a peptide antigen or a mixture of peptide antigens).

In the disclosed methods, the disclosed compositions and formulations may be administered to a subject in order to stimulate T cell immunity. For example, the disclosed vaccine compositions may be administered to a subject in order to stimulate T cell immunity against infection by a pathogen. In some embodiments, the disclosed compositions and formulations may be administered to a subject in order to stimulate a Th1 cell immune response.

The present inventors have observed when the disclosed compositions and formulations comprising biodegradable particles are administered to a subject, the subject gains weight at higher relative rate than a subject that has not been administered the compositions and formulations. Therefore, the disclosed methods include methods of administering the disclosed compositions and formulations for inducing weight gain in a subject. The disclosed methods also include methods of administering the disclosed compositions and formulations to a subject for increasing feed conversion rate in the subject. The disclosed methods also include methods of administering the disclosed compositions and formulations to a subject for increasing survival rate. Suitable subjects for the methods for inducing weight gain and/or for increasing feed conversion rate may include, but are not limited to, fowl ,such as chickens and turkeys, swine, and ruminants, such as cattle, sheep, and goats.

### BRIEF DESCRIPTION OF THE DRAWINGS

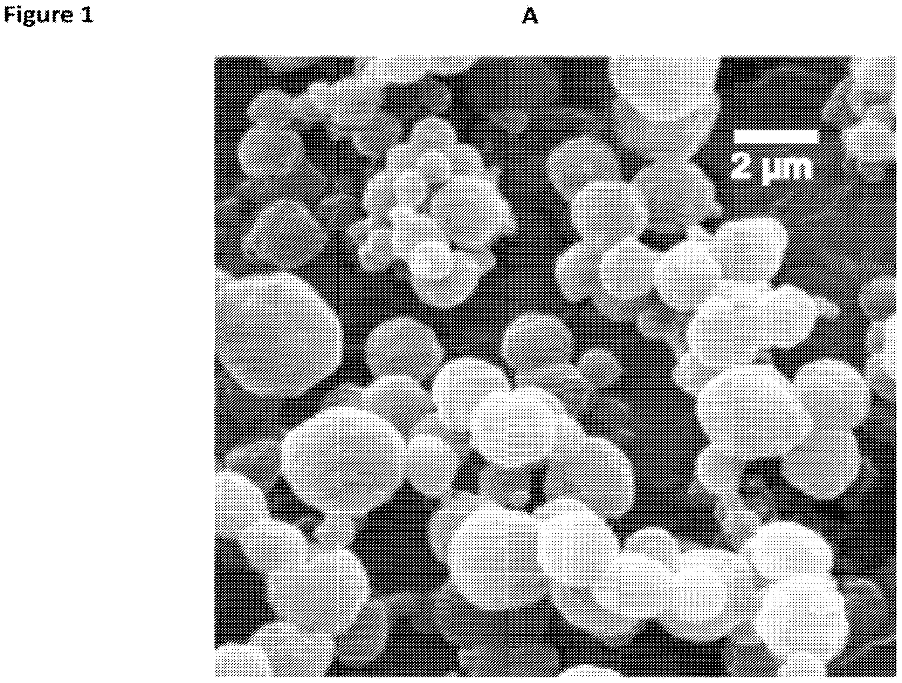

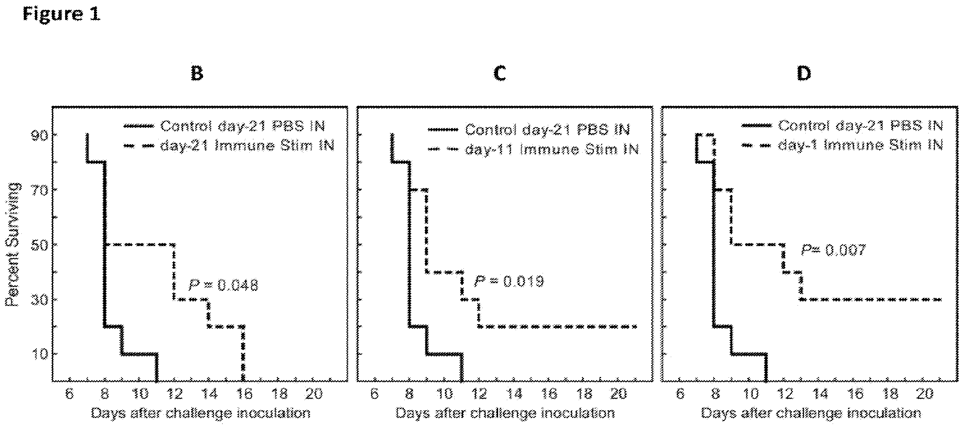

FIG. 1. A. Scanning electron micrograph of spray-dried microparticles composed of PLGA-PEG:Pluronic L121 = 3:2. B. Percent survival when challenged day 21 post-administration of immune stimulator. C. Percent survival when challenged day 11 post-administration of immune stimulator. D. Percent survival when challenged day 1 post-administration of immune stimulator.

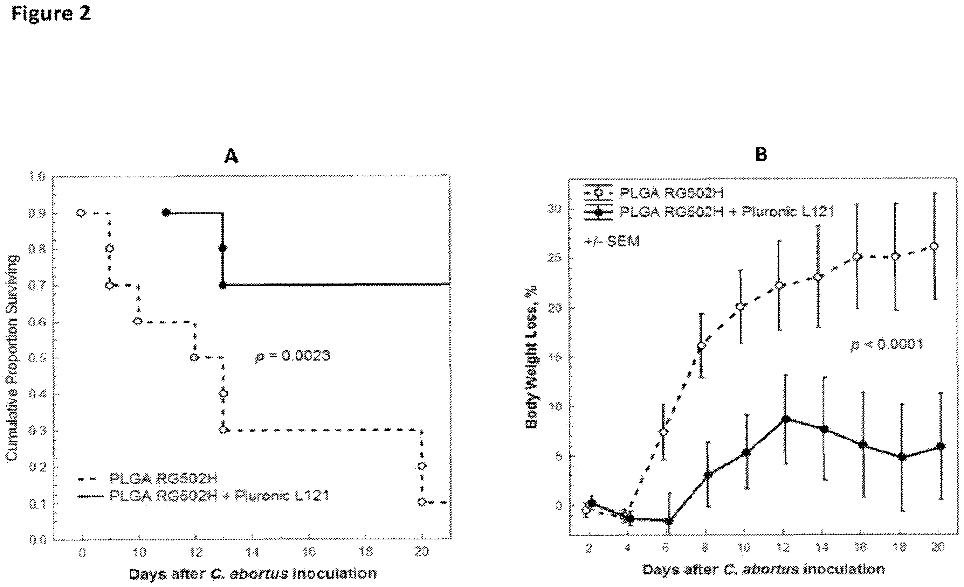

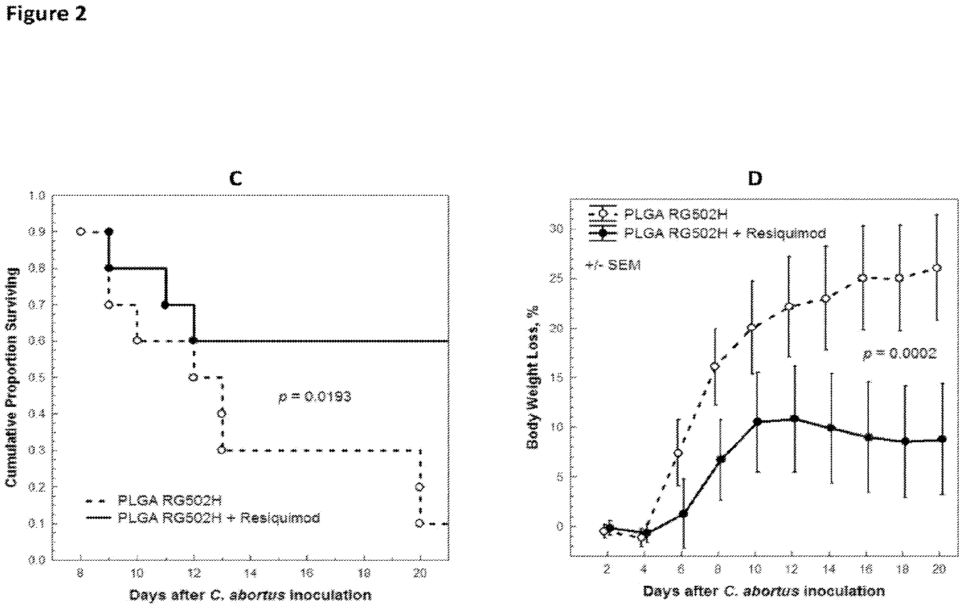

FIG. 2. A. Cumulative proportion surviving versus days after challenge with *C*. *abortus* for mice administered PLGA RG502H particle composition + Pluronic L121® versus PLGA RG502H composition without Pluronic L121®. B. Body weight loss versus days after challenge with *C*. *abortus* for mice administered PLGA RG502H particle composition + Pluronic L121® versus PLGA RG502H composition without Pluronic L121®. C. Cumulative proportion surviving versus days after challenge with *C*. *abortus* for mice administered PLGA RG502H composition + Resiquimod versus PLGA RG502H particle composition without Pluronic L121®. C. Body weight loss versus days after challenge with *C*. *abortus* for mice administered PLGA RG502H composition + Resiquimod versus PLGA RG502H particle composition without Pluronic L121®.

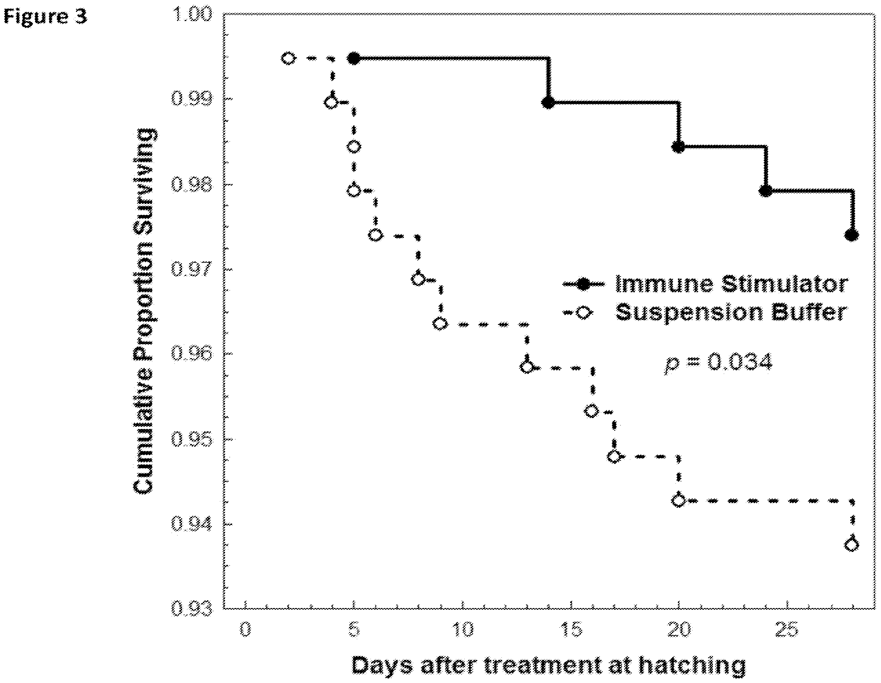

FIG. 3. Cumulative proportion surviving versus days after treatment at hatching for chickens administered immune stimulator versus buffer control.

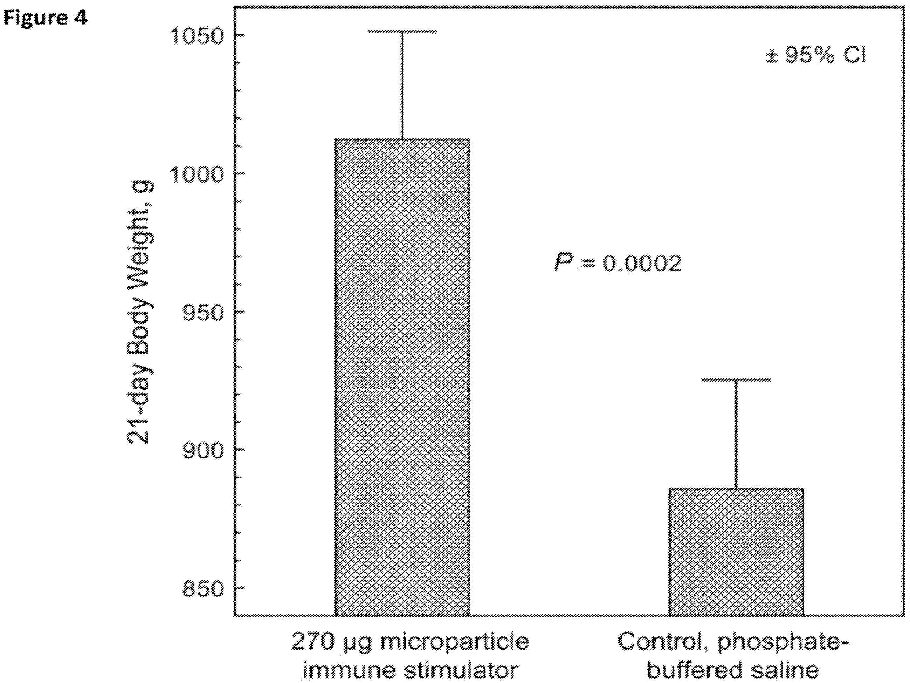

FIG. 4. Body weight after 21 days for chickens administered immune stimulator versus buffer control.

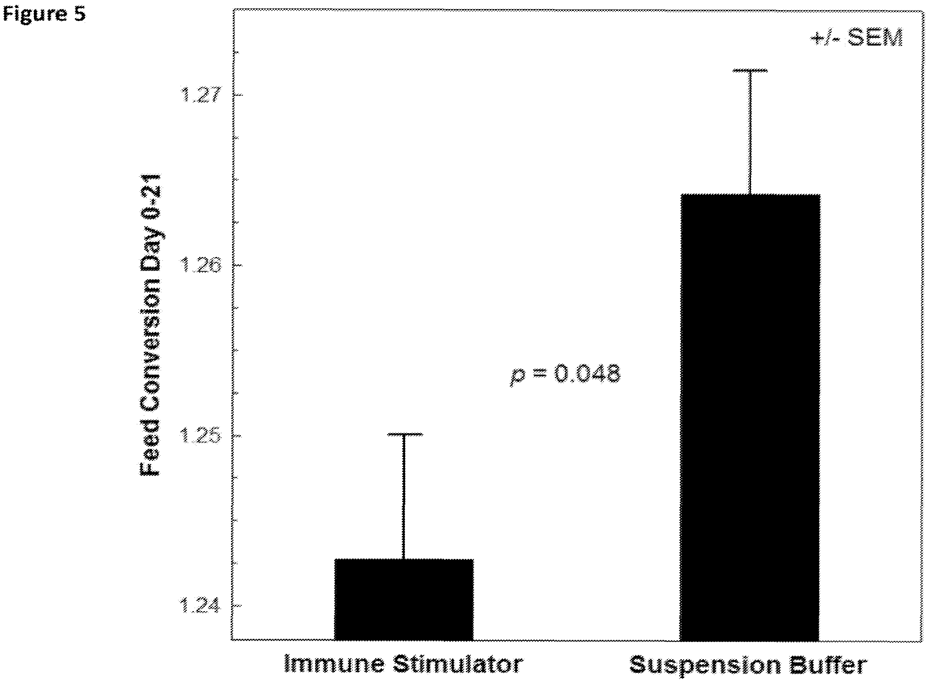

FIG. 5. Feed conversion rate from day 0 to day 21 for chickens administered immune stimulator versus buffer control.

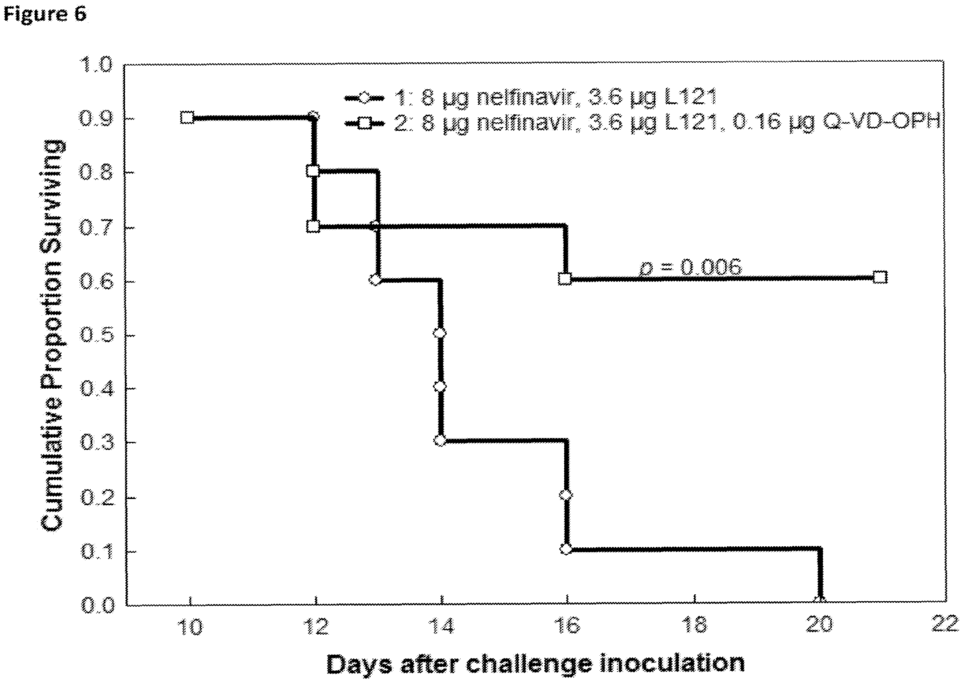

FIG. 6. Cumulative proportion surviving versus days after challenge with *C*. *abortus* for mice administered nelfinavir particle composition and Pluronic L121®, with or without apoptosis inhibitor Q-VD-OPH.

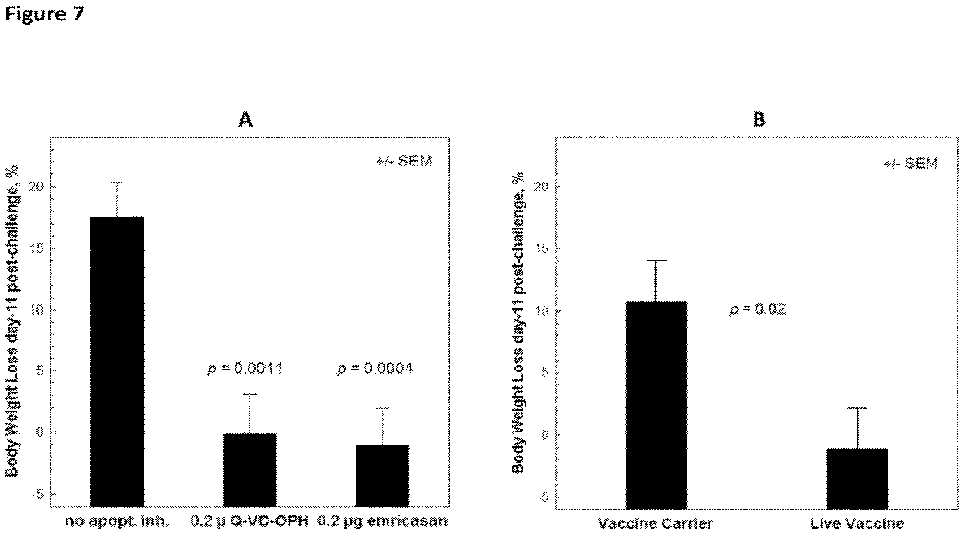

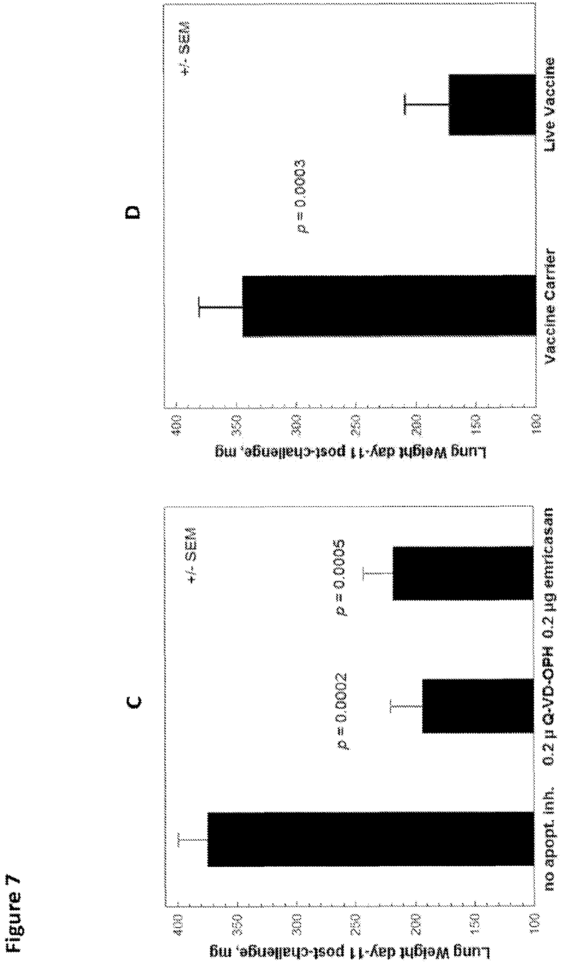

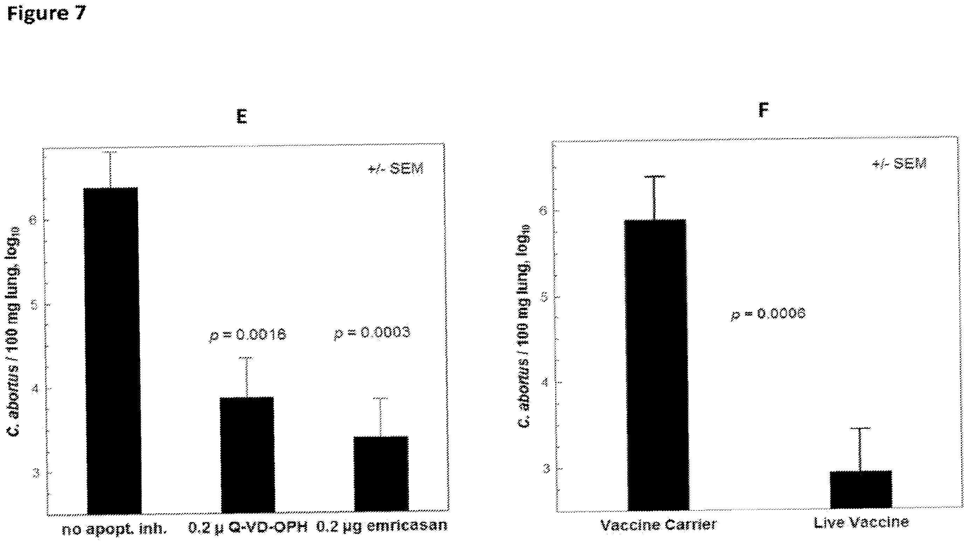

FIG. 7. A. Percent body weight loss at day 11 post-challenge for mice administered vaccine with apoptosis inhibitor Q-VD-OPH versus vaccine without apoptosis inhibitor. B. Percent body weight loss at day 11 post-challenge for mice administered vaccine carrier versus live vaccine. C. Lung weight at day 11 post-challenge for mice administered vaccine with apoptosis inhibitor Q-VD-OPH versus vaccine without apoptosis inhibitor. D. Lung weight at day 11 post-challenge for mice administered vaccine carrier versus live vaccine.

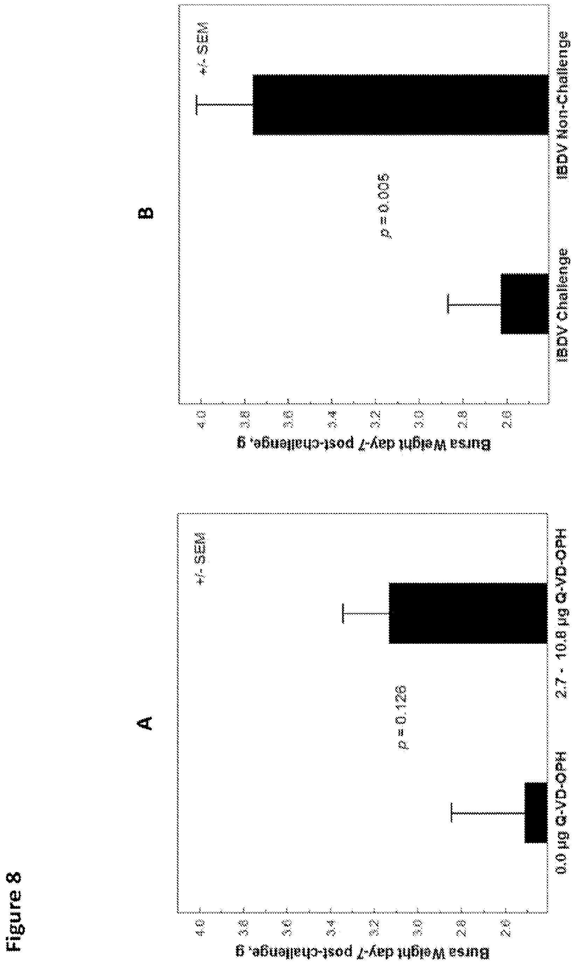

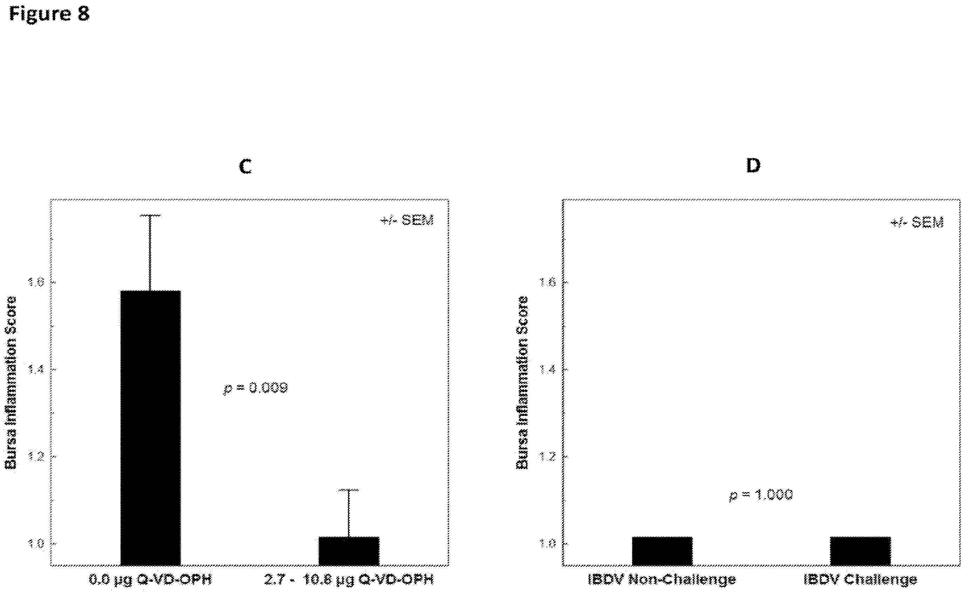

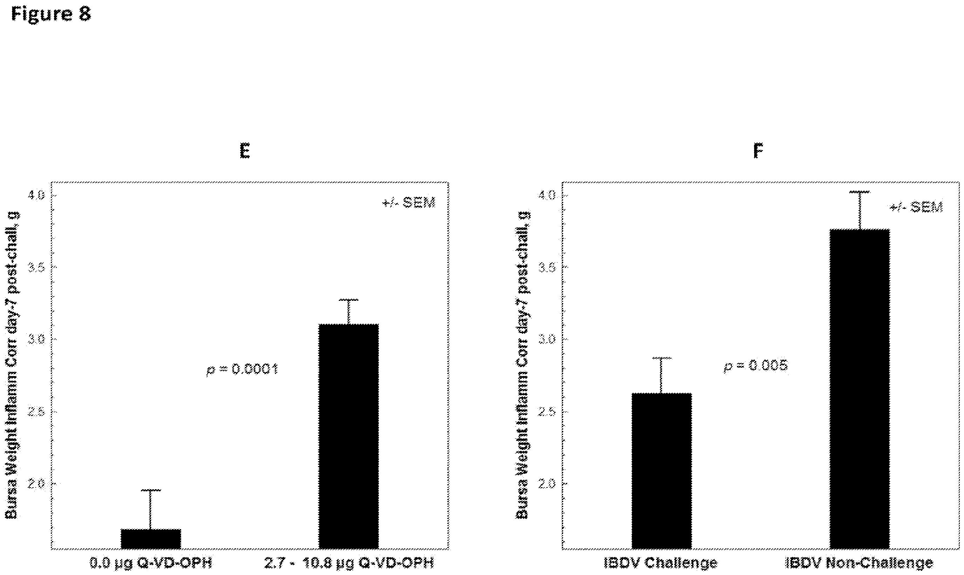

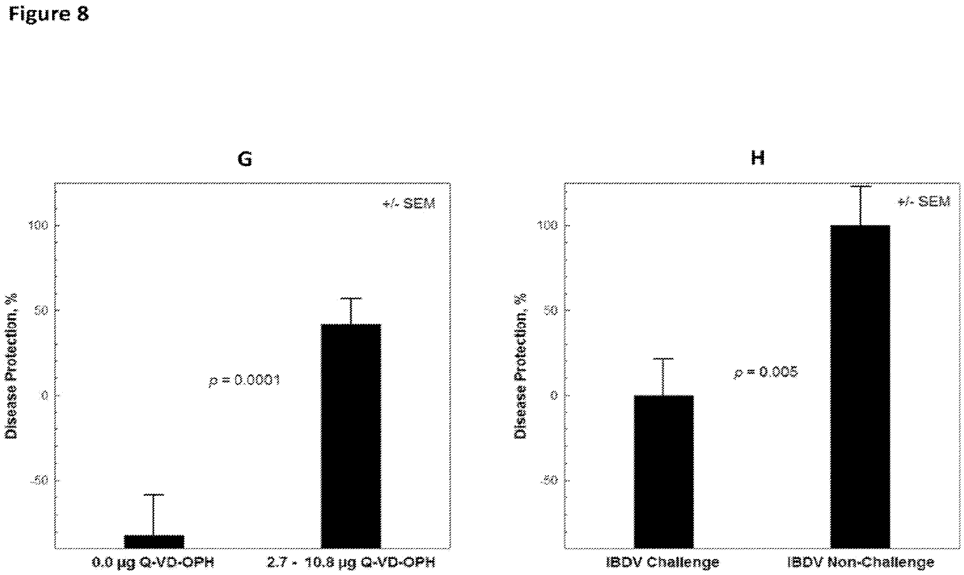

FIG. 8. A. Bursa of Fabricius weight at day 7 post-challenge with IBDV for chickens administered vaccine with apoptosis inhibitor Q-VD-OPH versus vaccine without apoptosis inhibitor. B. Bursa weight at day 7 post-challenge with IBDV for chickens administered suspension buffer control versus non-challenged chickens. C. Bursa inflammation score for challenged chickens administered vaccine with apoptosis inhibitor Q-VD-OPH versus vaccine without apoptosis inhibitor. D. Bursa inflammation score for challenged chickens administered suspension buffer control versus non-challenged chickens. E. Bursa weight at day 7 post-challenge with IBDV corrected for inflammation score for chickens administered vaccine with apoptosis inhibitor Q-VD-OPH versus vaccine without apoptosis inhibitor. F. Bursa weight at day 7 post-challenge with IBDV corrected for inflammation score for chickens administered suspension buffer control versus non-challenged chickens. G. Percent disease protection for chickens administered vaccine with apoptosis inhibitor Q-VD-OPH versus vaccine without apoptosis inhibitor. H. Percent disease protection for chickens administered suspension buffer control versus non-challenged chickens.

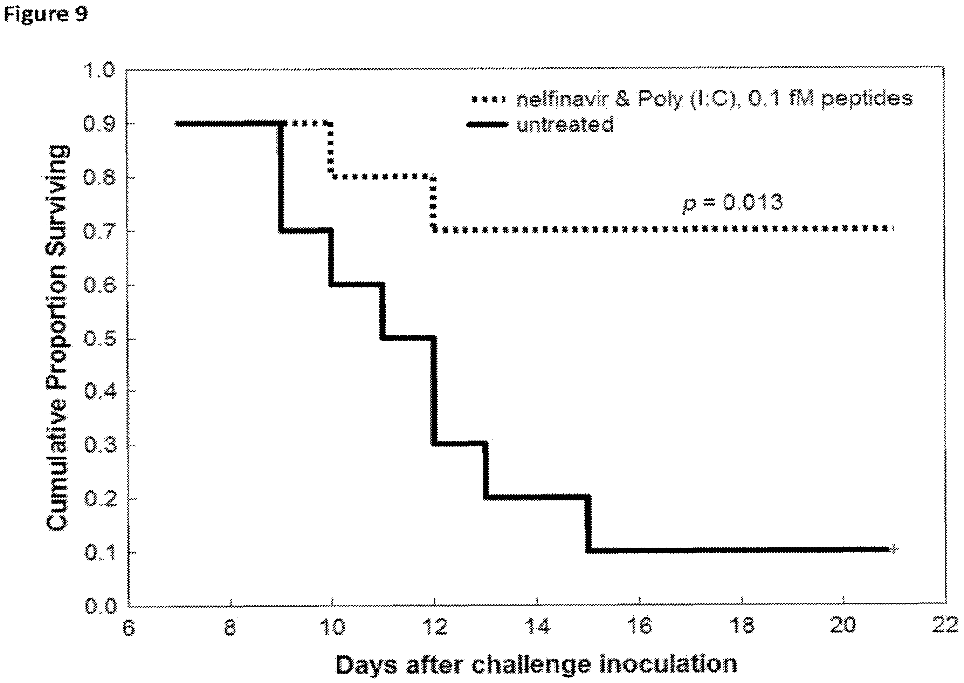

FIG. 9. Cumulative proportion surviving versus days after challenge with *C*. *abortus* for mice administered nelfinavir particle composition, Poly (I:C), and 1 fmole *C. abortus* peptides versus untreated.

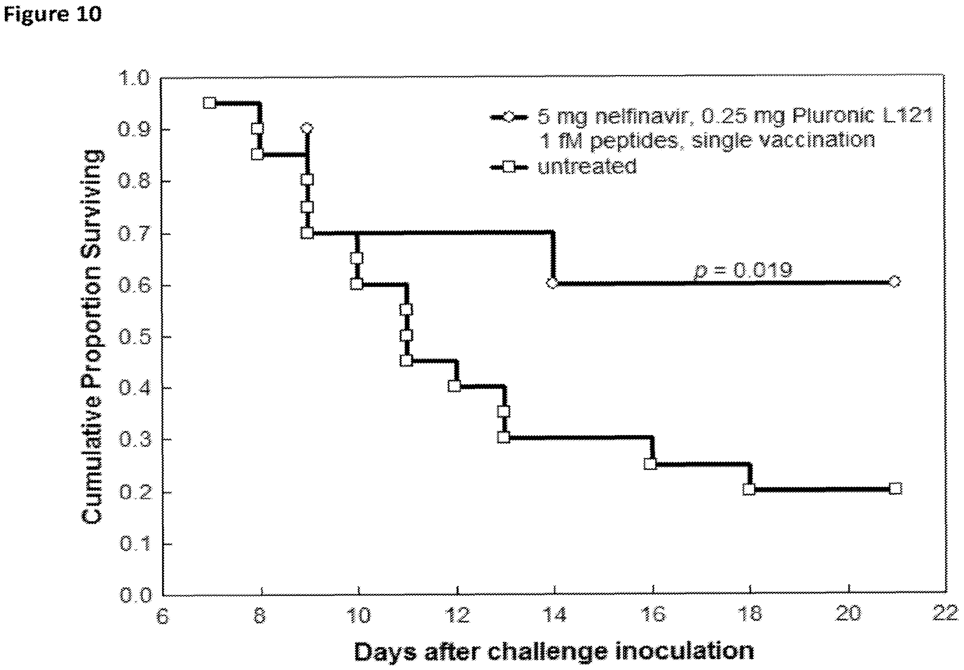

FIG. 10. Cumulative proportion surviving versus days after challenge with *C*. *abortus* for mice administered nelfinavir particle composition, Pluronic L121®, and 1 fmole *C*. *abortus* peptides versus untreated.

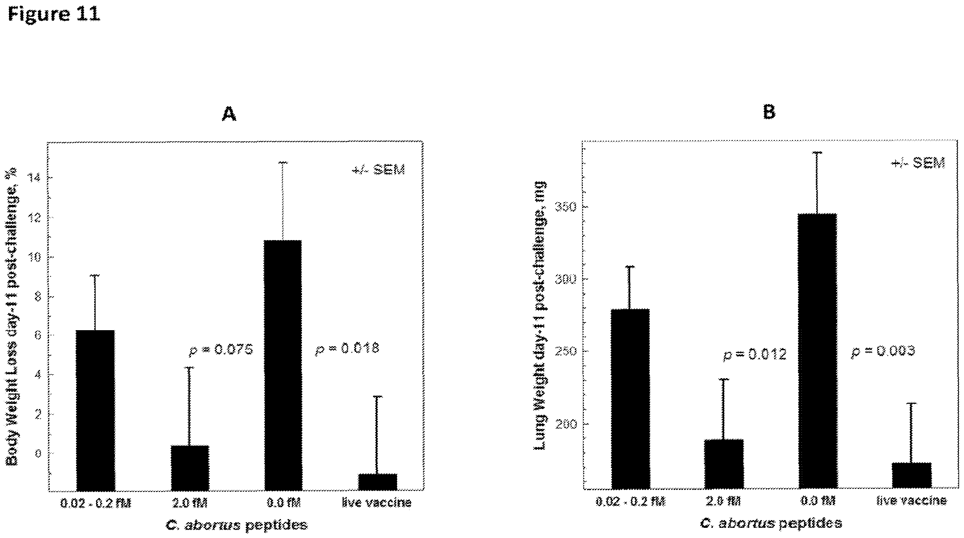

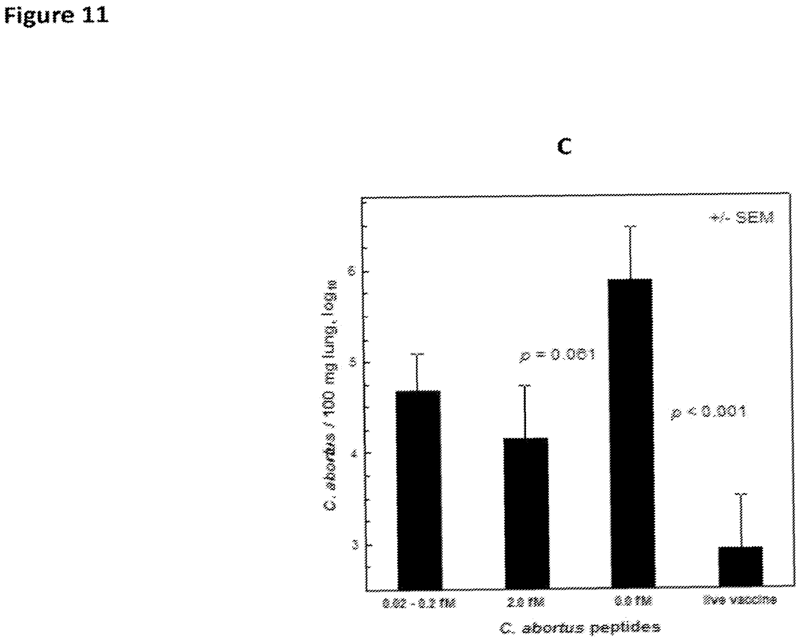

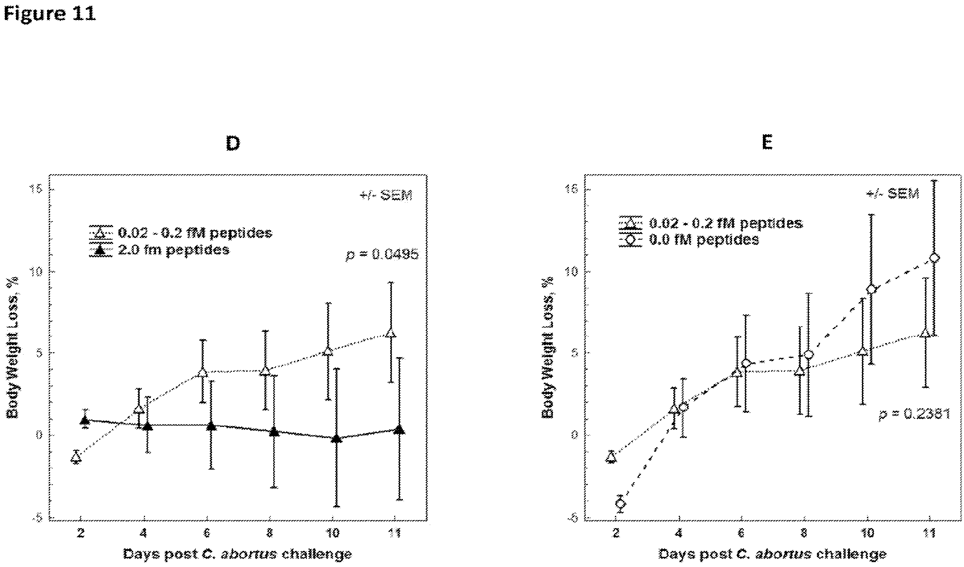

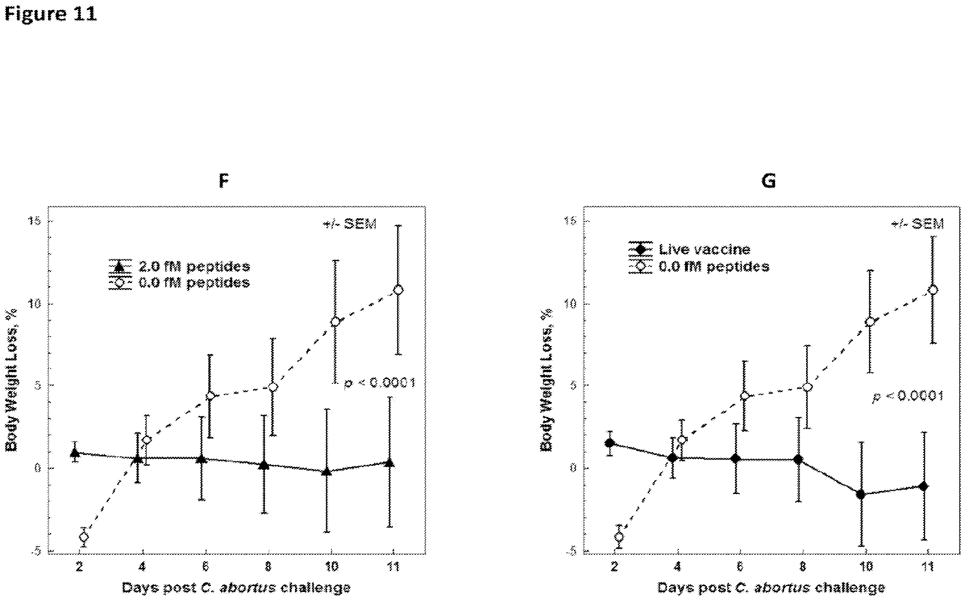

FIG. 11. A. Percent body weight loss at day 11 post-challenge for mice administered vaccine comprising 0.0, 0.02-0.2, or 2.0 femtomoles overlapping peptides from 5 protective proteins of *C*. *abortus* and live vaccine. B. Lung weight at day 11 post-challenged for mice administered vaccine comprising 0.0, 0.02-0.2, or 2.0 femtomoles overlapping peptides from 5 protective proteins of *C*. *abortus* and live vaccine. C. *C abortus* loads for mice administered vaccine comprising 0.0, 0.02-0.2, or 2.0 femtomoles overlapping peptides from 5 protective proteins of *C*. *abortus* and live vaccine. D. Percent body weight loss versus days post-challenge for mice administered vaccine comprising 0.02-0.2 or 2.0 femtomoles overlapping peptides from 5 protective proteins of *C*. *abortus.* E. Percent body weight loss versus days post-challenge for mice administered vaccine comprising 0.0 or 0.02-0.2 femtomoles overlapping peptides from 5 protective proteins of *C*. *abortus.* F. Percent body weight loss versus days post-challenge for mice administered vaccine comprising 0.0 or 2.0 femtomoles overlapping peptides from 5 protective proteins of *C*. *abortus.* G. Percent body weight loss versus days post-challenge for mice administered vaccine comprising 0.0 femtomoles overlapping peptides from 5 protective proteins of C. *abortus* and live vaccine.

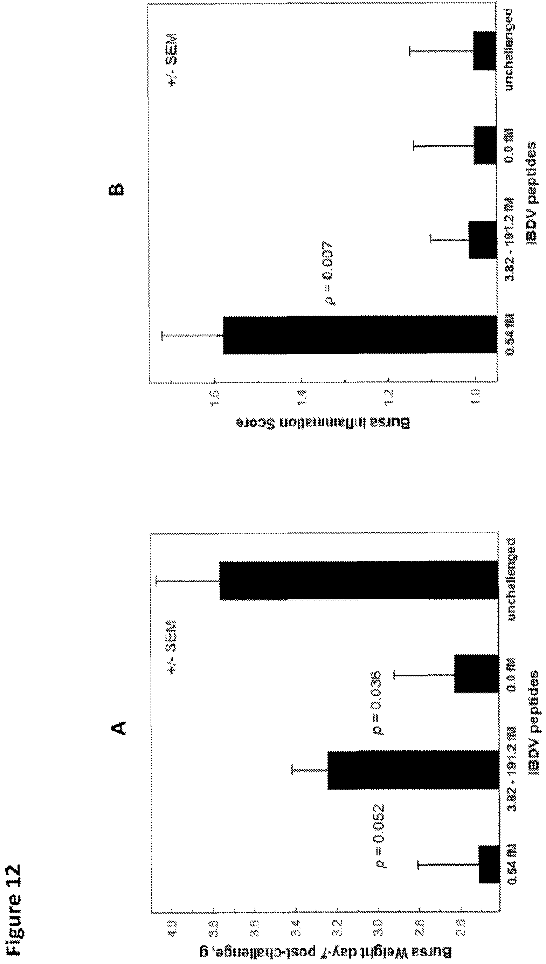

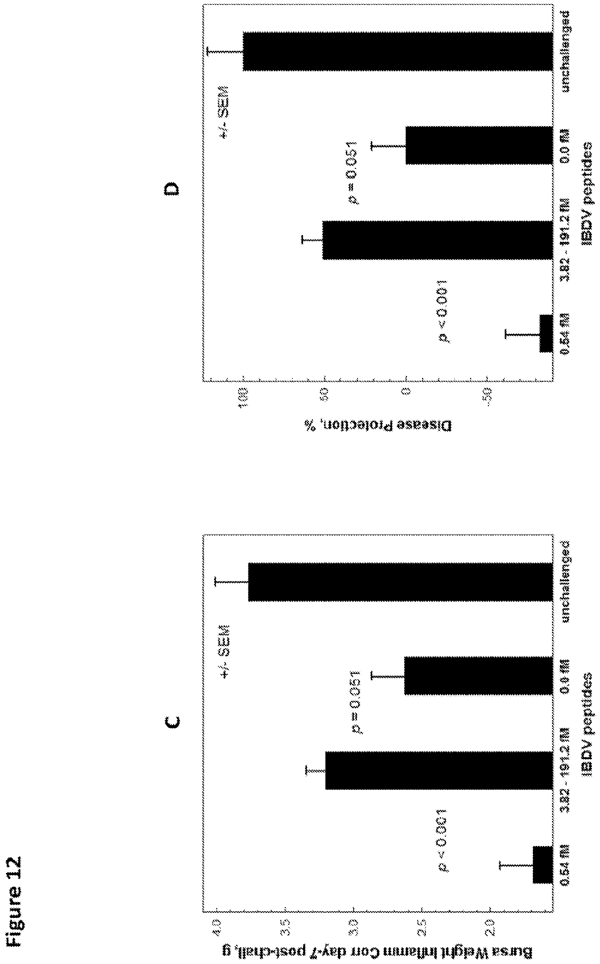

FIG. 12. A. Bursa of Fabricius weight at day 7 post-challenge for chickens administered vaccine comprising 0.0, 0.54, or 3.82-191.2 fmoles IBDV peptides versus unchallenged chickens. B. Bursa inflammation score at day 7 post-challenge for chickens administered vaccine comprising 0.0, 0.54, or 3.82-191.2 fmoles IBDV peptides versus unchallenged chickens. C. Bursa weight at day 7 post-challenge with IBDV corrected for inflammation score for chickens administered vaccine comprising 0.0, 0.54, or 3.82-191.2 fmoles IBDV peptides versus unchallenged chickens. D. Percent disease protection for chickens administered vaccine comprising 0.0, 0.54, or 3.82-191.2 fmoles IBDV peptides versus unchallenged chickens.

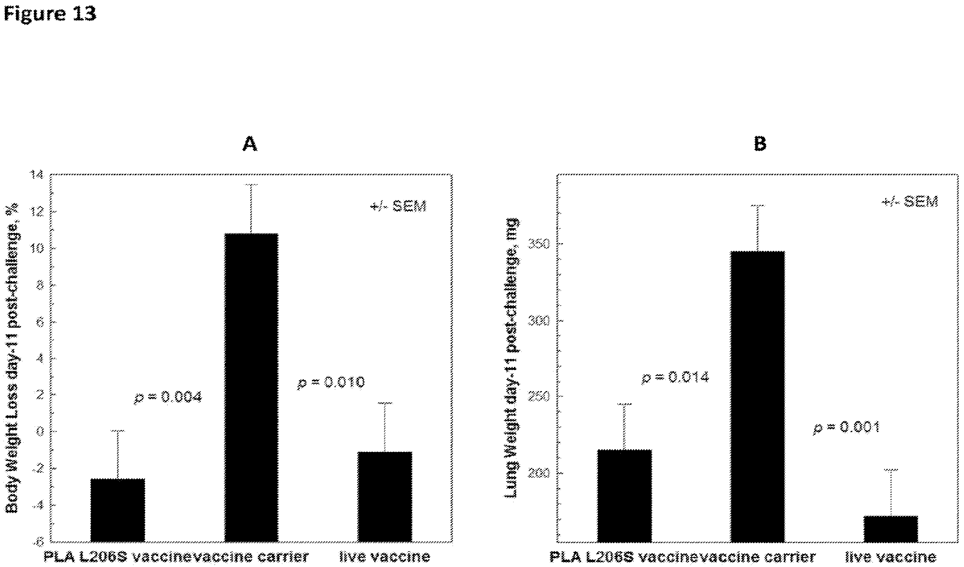

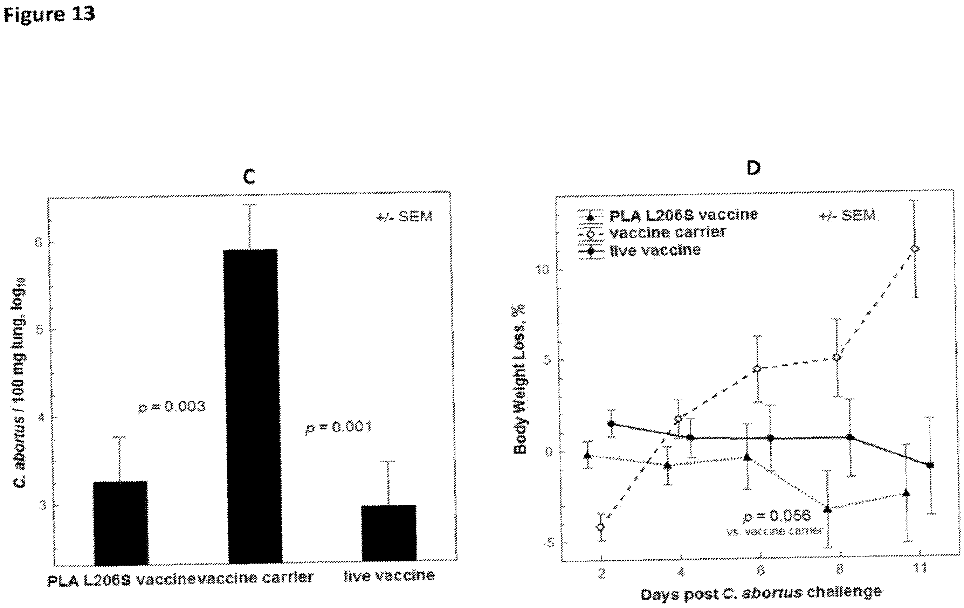

FIG. 13. A. Percent body weight loss at day 11 post-challenge for mice administered vaccine comprising 2.0 femtomoles overlapping peptides from 5 protective proteins of *C*. *abortus* and live vaccine. B. Lung weight at day 11 post-challenged for mice administered vaccine comprising 2.0 femtomoles overlapping peptides from 5 protective proteins of *C*. *abortus* and live vaccine. C. *C abortus* loads for mice administered vaccine comprising 2.0 femtomoles overlapping peptides from 5 protective proteins of *C*. *abortus* and live vaccine. D. Percent body weight loss versus days post-challenge for mice administered vaccine comprising 2.0 femtomoles overlapping peptides from 5 protective proteins of *C*. *abortus* and live vaccine.

### DETAILED DESCRIPTION

Disclosed herein are compositions, kits, and methods for inducing an immune response against disease which may be described using several definitions as discussed below.

Unless otherwise specified or indicated by context, the terms "a", "an", and "the" mean "one or more." In addition, singular nouns such as "adjuvant," "apoptosis inhibitor," and "antigen" should be interpreted to mean "one or more adjuvants," "one or more apoptosis inhibitors," and "one or more antigens," respectively, unless otherwise specified or indicated by context.

As used herein, "about", "approximately," "substantially," and "significantly" will be understood by persons of ordinary skill in the art and will vary to some extent on the context in which they are used. If there are uses of the term which are not clear to persons of ordinary skill in the art given the context in which it is used, "about" and "approximately" will mean plus or minus ≤10% of the particular term and "substantially" and "significantly" will mean plus or minus > 10% of the particular term.

As used herein, the terms "include" and "including" have the same meaning as the terms "comprise" and "comprising." The terms "comprise" and "comprising" should be interpreted as being "open" transitional terms that permit the inclusion of additional components further to those components recited in the claims. The terms "consist" and "consisting of" should be interpreted as being "closed" transitional terms that do not permit the inclusion additional components other than the components recited in the claims. The term "consisting essentially of" should be interpreted to be partially closed and allowing the inclusion only of additional components that do not fundamentally alter the nature of the claimed subject matter.

The terms "subject," "patient," or "host" may be used interchangeably herein and may refer to human or non-human animals. Non-human animals may include, but are not limited to fowl (*e.g*., chickens and turkeys), cows, pigs, horses, dogs, and cats.

The terms "subject," "patient," or "individual" may be used to a human or non-human animal having or at risk for acquiring infection by a pathogen (*e.g.* a bacterial, viral, or fungal pathogen) or a disease (*e.g*., cancer or an autoimmune disease) that is amenable to treatment or protection by a vaccine. For example, individuals who are treated with the compositions disclosed herein may be at risk for infection with a pathogen or may have already been infected with the pathogen. Individuals who are treated with the compositions disclosed herein may be at risk for cancer or may have already acquired cancer. Individuals who are treated with the compositions disclosed herein may be at risk for an autoimmune disease or may have already acquired an autoimmune disease.

Biodegradable Particles. The disclosed compositions include compositions comprising biodegradable particles. The biodegradable particles typically have an effective average diameter of 0.1-20 µm and preferably 0.5-10 µm. The biodegradable particles may be referred to herein as "microparticles" and/or "nanoparticles." The particles are biodegradable as would be understood in the art. The term "biodegradable" describes a material that is capable of being degraded in a physiological environment into smaller basic components. Preferably, the smaller basic components are innocuous. For example, an biodegradable polymer may be degraded into basic components that include, but are not limited to, water, carbon dioxide, sugars, organic acids (*e.g.,* tricarboxylic or amino acids), and alcohols (*e.g.,* glycerol or polyethylene glycol). Biodegradable materials that may be utilized to prepare the particles contemplated herein may include materials disclosed in U.S. Patent Nos. 7,470,283; 7,390,333; 7,128,755; 7,094,260; 6,830,747; 6,709,452; 6,699,272; 6,527,801; 5,980,551; 5,788,979; 5,766,710; 5,670,161; and 5,443,458; and U.S. Published Application Nos. 20090319041; 20090299465; 20090232863; 20090192588; 20090182415; 20090182404; 20090171455; 20090149568; 20090117039; 20090110713; 20090105352; 20090082853; 20090081270; 20090004243; 20080249633; 20080243240; 20080233169; 20080233168; 20080220048; 20080154351; 20080152690; 20080119927; 20080103583; 20080091262; 20080071357; 20080069858; 20080051880; 20080008735; 20070298066; 20070288088; 20070287987; 20070281117; 20070275033; 20070264307; 20070237803; 20070224247; 20070224244; 20070224234; 20070219626; 20070203564; 20070196423; 20070141100; 20070129793; 20070129790; 20070123973; 20070106371; 20070050018; 20070043434; 20070043433; 20070014831; 20070005130; 20060287710; 20060286138; 20060264531; 20060198868; 20060193892; 20060147491; 20060051394; 20060018948; 20060009839; 20060002979; 20050283224; 20050278015; 20050267565; 20050232971; 20050177246; 20050169968; 20050019404; 20050010280; 20040260386; 20040230316; 20030153972; 20030153971; 20030144730; 20030118692; 20030109647; 20030105518; 20030105245; 20030097173; 20030045924; 20030027940; 20020183830; 20020143388; 20020082610; and 0020019661; the contents of which are incorporated herein by reference in their entireties. Typically, the biodegradable particles disclosed herein are degraded *in vivo* at a degradation rate such that the particles lose greater than about 50%, 60%, 70%, 80%, 90%, 95%, or 99% of their initial mass after about 4, 5, 6, 7, or 8 weeks post-administration. The particles may comprise or may be formed from polymeric or non-polymeric biodegradable material. If the particles comprise polymeric material, typically the particles are degraded into biodegradable monomers. If the particles comprise non-polymeric material, typically the particles are degraded into biodegradable components.

Suitable polymers for preparing the biodegradable particles may include, but are not limited to, polymers such as polylactides (PLA), including polylactic acid, for example, polyglycolides (PGA), including polyglycolic acid, and co-polymers of PLA and PGA (*i.e.,* PLGA). Other suitable polymers may include, but are not limited to,, polycaprolactone (PCL), poly(dioxanone) (PDO), collagen, renatured collagen, gelatin, renatured gelatin, crosslinked gelatin, and their co-polymers. The polymer of the biodegradable particles is designed to degrade as a result of hydrolysis of polymer chains into biologically acceptable and progressively smaller components such as polylactides, polyglycolides, and their copolymers. These break down eventually into lactic and glycolic acid, enter the Kreb's cycle and are broken down into carbon dioxide and water and excreted.

Suitable non-polymers may include poorly soluble compounds such as compounds shown to function as immunomodulators. One suitable compound is nelfinavir, which has been shown to exhibit an immunopotentiating effect. As such, biodegradable particles that are contemplated herein may include biodegradable particles formed from immunomodulating compounds.

The disclosed biodegradable particles may be prepared by methods known in the art including, but not limited to, spray-drying, precipitation, and grinding. In some embodiments, the biodegradable particles may be formed from a solution or suspension of a biodegradable material optionally in the presence of one or more additional agents such as adjuvants, apoptosis inhibitors, and/or antigens (*e.g*., by spray-drying the solution or suspension). As such, the biodegradable particles may comprise biodegradable material and optionally may comprise one or more additional agents such as adjuvants, apoptosis inhibitors, and/or antigens.

The disclosed biodegradable particles may be administered in order to induce a response in a subject. In some embodiments, the disclosed methods comprise administering a composition comprising biodegradable particles to induce an immune response in the subject. In other embodiments, the disclosed methods consist of administering a composition consisting of biodegradable particles to induce an immune response in the subject. The induced immune response may include a Th1 cell response. The induced immune response in a subject administered the composition may cause the subject to exhibit higher weight gain or better feed conversion rate than a subject that is not administered the composition. In some embodiments, the disclosed methods comprise administering a composition comprising biodegradable particles to induce weight gain in a subject and/or to improve feed conversion rate in a subject.

The dose of biodegradable particles administered in the disclosed methods may vary based on the weight of a subject. For example, a mouse having a weight of about 20 g may be administered a dose of particles equivalent to about 1 - 100 µg (or 2 - 50 µg or 5 - 20 µg). This dose may be allometrically scaled based on the formula (BW/20)3/4 = allometric scaling factor, where "BW" equals the body weight of the target animal in grams. Assuming that the target animal is a chicken weight 1600 g, the allometric scaling factor is (1,600/20)^{3/4} = 26.7. Multiplying the 10 µg microparticle amount used for a 20 g mouse by the scaling factor of 26.7 for a 1600 g chicken results in a microparticle dose of ∼270 µg. Similarly, for a human having a weight of 80,000 g, the allometric scaling factor is (80000/20)^{3/4} = ∼503. Multiplying the 10 µg microparticle amount used for a 20 g mouse by the scaling factor of 503 for a 80000 g human results in a microparticle dose of ∼5030 µg.

Adjuvants. The compositions disclosed herein optionally include an adjuvant. The term "adjuvant" refers to a compound or mixture that enhances an immune response. An adjuvant can serve as a tissue depot that slowly releases the antigen and also as a lymphoid system activator that non-specifically enhances the immune response. Examples of adjuvants which may be utilized in the disclosed compositions include but are not limited to, co-polymer adjuvants (*e.g*., Pluronic L121® brand poloxamer 401, CRL1005, or a low molecular weight co-polymer adjuvant such as Polygen® adjuvant), poly (I:C), R-848 (a Th1-like adjuvant), resiquimod, imiquimod, PAM3CYS, aluminum phosphates (*e.g*., AlPO₄), loxoribine, potentially useful human adjuvants such as BCG (Bacille Calmette-Guerin) and *Corynebacterium parvum,* CpG oligodeoxynucleotides (ODN), cholera toxin derived antigens (*e.g*., CTA1-DD), lipopolysaccharide adjuvants, complete Freund's adjuvant, incomplete Freund's adjuvant, saponin (*e.g*., Quil-A), mineral gels such as aluminum hydroxide, surface active substances such as lysolecithin, pluronic polyols, polyanions, peptides, oil or hydrocarbon emulsions in water (*e.g.,* MF59 available from Novartis Vaccines or Montanide ISA 720), keyhole limpet hemocyanins, and dinitrophenol.

Apoptosis Inhibitors. The compositions disclosed herein optionally may include an apoptosis inhibitor. An "apoptosis inhibitor" refers to a small molecule that inhibits a cell's initiation of or progression through the apoptosis process. Apoptosis inhibitors may include small inhibitors of pan-caspase (*e.g*., Q-VD-OPH and emriscan) or inhibitors of other enzymes involved in the apoptotic pathways, as well as inhibitors of c-Myc, Bax, p53, tBid, and BCL which mediate apoptosis.

Antigens and Dose. The compositions disclosed herein optionally may include an antigen, a panel of antigens, or a plurality of antigens. In embodiments of the disclosed compositions comprising biodegradable particles and antigens, the particles and antigens may be present in the compositions at a suitable ratio. For example, the particles and antigens may be present in a molar ratio of about 0.2, 0.5, 1.0, 2.0, or 5.0, and preferably at a ratio approaching 1.0.

The disclosed composition may comprise a "panel" or "plurality of antigens." A "panel" or "plurality" or antigens as used herein means "more than one" and may mean more than 1, 2, 3, 4, 5, 10, 25, 50, or 100 antigens. A panel or plurality of antigens may include a set of different, overlapping polypeptides (*e.g*., polypeptides of about 10-20 amino acids that overlap by about 5-10 amino acids) where the overlapping polypeptides correspond to a full-length polypeptide associated with a disease. A panel of polynucleotides may encode different or unique amino acid sequences of a selected polypeptide. The encoded different or unique amino acid sequences may overlap. For example, a panel of overlapping polypeptides may correspond to the full-length sequence of a protein where a first polypeptide of a panel includes amino acids 1-20 of the protein, the second polypeptide of the panel includes amino acids 11-30 of the protein, the third polypeptide of the panel includes amino acids 21-40 of the protein, the fourth polypeptide of the panel includes amino acids 31-50 of the protein, such the overlapping polypeptides of the panel encompass all of the amino acid sequence of the protein.

The composition, kits, and methods contain or utilize a protein, polypeptide, peptide, or panel thereof as an antigen. In some embodiments, the dosage of antigen contained or utilized in the presently disclosed compositions, kits, and methods is substantially lower than that dosage conventionally used in the field (*e.g.,* by at least an order of magnitude (10×)). The compositions, kits, and methods may be utilized to induce a cell-mediated response (*e.g*., a T-helper cell response) and/or a humoral response against a disease. In some embodiments, the compositions, kits, and methods may be utilized to induce preferentially a Th1 response versus other types of immune responses (*e.g*., a Th2 response).

In some embodiments, the disclosed compositions, kits, and methods include or utilize a relatively low amount of antigen compared to vaccines and methods of the art. As contemplated herein, suitable doses administered to a subject in need thereof may be no more than about 2 pg antigen / g body weight (preferably no more than about 1 pg antigen / g body weight, more preferably no more than about 0.5 pg antigen / g body weight, more preferably no more than about 0.2 pg antigen / g body weight, more preferably no more than about 0.1 pg antigen / g body weight, more preferably no more than about 0.05 pg antigen / g body weight, even more preferably no more than about 0.01 pg antigen / g body weight). In some embodiments, a suitable dose administered to a subject in need thereof may be at least about 0.01 pg antigen / g body weight, at least about 0.05 pg antigen / g body weight, or at least about 0.1 pg antigen / g body weight. For example, suitable dose ranges may include 0.01 - 0.05 pg antigen / g body weight, 0.01 - 0.1 pg antigen / g body weight, or 0.01 - 0.2 pg antigen / g body weight, 0.01 - 1 pg antigen / g body weight, 0.01 - 2 pg antigen / g body weight, 0.05 - 0.1 pg antigen / g body weight, 0.05 - 0.2 pg antigen / g body weight, 0.05 - 1 pg antigen / g body weight, or 0.05 - 2 pg antigen / g body weight, 0.1 - 0.2 pg antigen / g body weight, 0.1 - 1 pg antigen / g body weight, or 0.1 - 2 pg antigen / g body weight.

The compositions, kits, and methods disclosed herein may involve administering a peptide or a panel of peptides as an antigen in order to induce an immune response against a disease. For example, the compositions, kits, and methods disclosed herein may involve administering a peptide or a panel of peptides comprising 5 - 100 amino acids (preferably 10 - 20 amino acids). Typically, the peptides have a molecular weight of no more than about 5 kDa (preferably no more than about 4 kDa, more preferably no more than about 3 kDa). Suitable doses of the peptide or the panel of peptides administered to a subject in need thereof as described by moles administered per gram body weight of subject may be no more than about 1 femtomole each peptide / g body weight (preferably no more than about 0.5 femtomoles each peptide / g body weight, more preferably no more than about 0.1 femtomoles each peptide / g body weight, more preferably no more than about 0.05 femtomoles each peptide / g body weight, even more preferably no more than about 0.01 femtomoles each peptide / g body weight). In some embodiments, a suitable dose administered to a subject in need thereof as described by moles each peptide per gram body weight of subject may be at least about 0.01 femtomoles each peptide / g body weight, or at least about 0.05 femtomoles antigen / g body weight. For example, suitable dose ranges may include 0.01 - 0.05 femtomoles antigen / g body weight, 0.01 - 0.1 femtomoles antigen / g body weight, 0.01 - 0.5 femtomoles antigen / g body weight, include 0.01 - 1 femtomoles antigen / g body weight, 0.05 - 0.1 femtomoles antigen / g body weight 0.05 - 0.5 femtomoles antigen / g body weight, and 0.05 - 1 femtomoles antigen / g body weight.

The compositions, kits, and methods may include or utilize a relatively low amount of antigen to induce an immune response (*e.g*., a Th-1 response) compared to convention vaccines and methods of the art. (*See* U.S. Published Application No. 2012/0009220, the contents of which are incorporated herein by reference in their entirety). Conventional vaccines and methods typically involve administering at least about 3 µg of an antigen per dose to a subject. (*See, e.g.,* Scheifele et al. 2005, Hum. Vaccin. 1:180-186; Evans et al. 2001, Vaccine 19:2080-2091; and Kenney et al., N. Engl. J. Med. 351:2295-2301, the contents of which are incorporated herein by reference in their entireties). However, a dose as low as 1 µg of an antigen per dose to a subject also has been proposed. (*See* U.S. Patent No. 6,372,223, the content of which is incorporated herein by reference in its entirety). Assuming that the subject is human and weighs approximately 75 kg, a dose of 1 µg antigen translates to a dose of 13.3 pg antigen / g body weight. In some embodiments of the presently disclosed compositions, kits, and methods, a dose rate that is an order of magnitude lower (*e.g.,* no more than about 2 pg antigen / g body weight) can be administered in order to induce an immune response (*e.g*., a Th1-response). For peptide vaccines as contemplated herein, a dose rate of 1 femtomole each peptide / g body weight or lower can be administered in order to induce an immune response (*e.g*., a Th1-response). Vaccines that comprise an antigen solution typically have an antigen concentration of no more than about 1.5 × 10⁻⁶ g antigen / ml (preferably no more than about 1.5 × 10⁻⁷ g antigen / ml, more preferably no more than about 1.5 × 10⁻⁸ g antigen / ml, more preferably no more than 1.5 × 10⁻⁹ g antigen / ml, even more preferably no more than about 1.5 × 10⁻¹⁰ g antigen / ml). In some embodiments, the vaccines comprise an antigen solution having an antigen concentration of at least about 1.5 × 10⁻¹⁰ g antigen / ml. For example, suitable concentration ranges may include 1.5 × 10⁻¹⁰ - 3 × 10⁻¹⁰ g antigen / ml, 1.5 × 10⁻¹⁰ - 6 × 10⁻¹⁰ g antigen / ml, 1.5 × 10⁻¹⁰ - 1.5 × 10⁻⁹ g antigen / ml, 1.5 × 10⁻¹⁰ - 3 × 10⁻⁹ g antigen / ml, or 1.5 × 10⁻¹⁰ - 6 × 10⁻⁹ g antigen / ml.

The vaccines disclosed herein may comprise a peptide or a panel of peptides as an antigen. For example, the vaccines may comprise a peptide or a panel of peptides comprising 5 - 100 amino acids (preferably 10 - 20 amino acids). Typically, the peptides have a molecular weight of no more than about 5 kDa (preferably no more than about 4 kDa, more preferably no more than about 3 kDa). Vaccines that comprise a peptide or a panel of peptides in solution typically have a solution concentration of each peptide of no more than about 7.5 × 10⁻¹⁰ moles each peptide /ml (preferably no more than about 1.5 × 10⁻¹¹ moles each peptide / ml, more preferably no more than about 7.5 × 10⁻¹² moles each peptide / ml, more preferably no more than about 1.5 × 10⁻¹² moles each peptide / ml, more preferably no more than about 7.5 × 10⁻¹³ moles each peptide / ml, even more preferably no more than about 1.5 × 10⁻¹³ moles each peptide / ml). In some embodiments, the vaccines comprise a peptide solution having a concentration of at least about 1.5 × 10⁻¹³ moles each peptide / ml, or at least about 1.5 × 10⁻¹³ moles each peptide / ml. For example, suitable concentration ranges may include 1.5 × 10⁻¹³ - 3 × 10⁻¹³ moles each peptide / ml, 1.5 × 10⁻¹³ - 6 × 10⁻¹³ moles each peptide / ml, 1.5 × 10⁻¹³ - 1.5 × 10⁻¹² moles each peptide / ml, 1.5 × 10⁻¹³ - 3 × 10⁻¹² moles each peptide / ml, 3 × 10⁻¹³ - 6 × 10⁻¹³ moles each peptide / ml, 3 × 10⁻¹³ - 1.5 × 10⁻¹² moles each peptide / ml, 3 × 10⁻¹³ - 3 × 10⁻¹² moles each peptide / ml.

Suitable antigens may include polypeptides, peptides, or panels thereof that comprise one or more epitopes of a protein associated with a disease. For example suitable polypeptides, peptides, or panels thereof may comprise one or more epitopes of a protein associated with a pathogen. Suitable polypeptides may comprise the full-length amino acid sequence of a corresponding protein of a pathogen or a fragment thereof. For example, suitable fragments may include 5-200 amino acids (or from 5-150, 5-100, 5-50, 5-25, 5-15, 10-200, 10-100, 10-50, 10-25, 10-25, or 10-15 amino acids) and include at least one epitope of the protein from which the fragment is derived. Suitable antigens for the compositions, kits, and methods may include panels of peptides derived from a protein of a pathogen. For example, a suitable antigen may comprise a panel of at least 2, 3, 4, 5, 10, 25, 50, 100, or more different peptides comprising at least about a 10 - 20 amino acid sequence from a protein of a pathogen. The different peptide antigens may overlap at the N-terminus, the C-terminus, or both termini with at least one other peptide antigen of the composition, for example, by at least about 1, 2, 3, 4, 5, 6, 7, 8, 9, or 10 amino acids.

Nature of Protein, Polypeptide, or Peptide Antigens. The presently disclosed compositions, kits, and methods contain and/or utilize a protein, polypeptide, or peptide for inducing an immune response. However, the presently disclosed compositions, kits, and methods are distinguished from live vaccines or inactivated vaccines in that the protein, polypeptide, or peptide of the compositions, kits, and methods is isolated, purified, recombinant, or synthesized in vitro (*e.g*., chemically synthesized). For example, the compositions, kits, and methods contain and/or utilize a protein, polypeptide, or peptide that is recombinant, expressed in a host cell, and isolated or purified. In another example, the compositions, kits, and methods may contain a panel of polypeptides or peptides that are chemically synthesized (*e.g*., using liquid phase synthesis, or solid phase synthesis such as Fmoc solid phase synthesis or *t*-boc solid phase synthesis).

As utilized herein, a protein, polypeptide, and peptide refer to a molecule comprising a chain of amino acid residues joined by amide linkages. The term "amino acid residue," includes but is not limited to amino acid residues contained in the group consisting of alanine (Ala or A), cysteine (Cys or C), aspartic acid (Asp or D), glutamic acid (Glu or E), phenylalanine (Phe or F), glycine (Gly or G), histidine (His or H), isoleucine (Ile or I), lysine (Lys or K), leucine (Leu or L), methionine (Met or M), asparagine (Asn or N), proline (Pro or P), glutamine (Gln or Q), arginine (Arg or R), serine (Ser or S), threonine (Thr or T), valine (Val or V), tryptophan (Trp or W), and tyrosine (Tyr or Y) residues. The term "amino acid residue" also may include amino acid residues contained in the group consisting of homocysteine, 2-Aminoadipic acid, N-Ethylasparagine, 3-Aminoadipic acid, Hydroxylysine, β-alanine, β-Amino-propionic acid, allo-Hydroxylysine acid, 2-Aminobutyric acid, 3-Hydroxyproline, 4-Aminobutyric acid, 4-Hydroxyproline, piperidinic acid, 6-Aminocaproic acid, Isodesmosine, 2-Aminoheptanoic acid, allo-Isoleucine, 2-Aminoisobutyric acid, N-Methylglycine, sarcosine, 3-Aminoisobutyric acid, N-Methylisoleucine, 2-Aminopimelic acid, 6-N-Methyllysine, 2,4-Diaminobutyric acid, N-Methylvaline, Desmosine, Norvaline, 2,2'-Diaminopimelic acid, Norleucine, 2,3-Diaminopropionic acid, Ornithine, and N-Ethylglycine.

The terms "protein," "polypeptide," and "peptide" may be referred to interchangeably herein. However, the terms may be distinguished as follows. A "protein" typically refers to the end product of transcription, translation, and post-translation modifications in a cell. Accordingly, a protein typically exhibits a biological function. A polypeptide is typically an amino acid chain of length ≥ 100 amino acids (Garrett & Grisham, Biochemistry, 2nd edition, 1999, Brooks/Cole, 110, which is incorporated herein by reference in its entirety). A polypeptide, as contemplated herein, may comprise, but is not limited to, 100, 101, 102, 103, 104, 105, about 110, about 120, about 130, about 140, about 150, about 160, about 170, about 180, about 190, about 200, about 210, about 220, about 230, about 240, about 250, about 275, about 300, about 325, about 350, about 375, about 400, about 425, about 450, about 475, about 500, about 525, about 550, about 575, about 600, about 625, about 650, about 675, about 700, about 725, about 750, about 775, about 800, about 825, about 850, about 875, about 900, about 925, about 950, about 975, about 1000, about 1100, about 1200, about 1300, about 1400, about 1500, about 1750, about 2000, about 2250, about 2500 or more amino acid residues. A peptide, in contrast to a polypeptide, typically is a short polymer of amino acids, of a length typically of 20 or less amino acids (Garrett & Grisham, Biochemistry, 2nd edition, 1999, Brooks/Cole, 110, which is incorporated herein by reference in its entirety). In some embodiments, a peptide as contemplated herein may include no more than about 10, 11, 12, 13, 14, 15, 16, 17, 18, 19, or 20 amino acids.

Polypeptides and peptides as contemplated herein may be further modified to include non-amino acid moieties. Modifications may include but are not limited to acylation (*e.g.,* O-acylation (esters), N-acylation (amides), S-acylation (thioesters)), acetylation (*e.g.,* the addition of an acetyl group, either at the N-terminus of the protein or at lysine residues), formylation lipoylation (*e.g.,* attachment of a lipoate, a C8 functional group), myristoylation *(e.g.,* attachment of myristate, a C14 saturated acid), palmitoylation *(e.g.,* attachment of palmitate, a C16 saturated acid), alkylation (*e.g.,* the addition of an alkyl group, such as an methyl at a lysine or arginine residue), isoprenylation or prenylation (*e.g.,* the addition of an isoprenoid group such as farnesol or geranylgeraniol), amidation at C-terminus, glycosylation *(e.g.,* the addition of a glycosyl group to either asparagine, hydroxylysine, serine, or threonine, resulting in a glycoprotein), polysialylation (*e.g.,* the addition of polysialic acid), glypiation (*e.g.,* glycosylphosphatidylinositol (GPI) anchor formation, hydroxylation, iodination (*e.g.,* of thyroid hormones), and phosphorylation (*e.g.,* the addition of a phosphate group, usually to serine, tyrosine, threonine or histidine).

A "fragment" of a protein or a polypeptide as contemplated herein refers to a contiguous portion of the amino acid sequence of the protein or polypeptide. A fragment of a protein or polypeptide refers to less than a full-length amino acid sequence of the protein or polypeptide (*e.g.,* where the full-length amino acid sequence is truncated at the N-terminus, the C-terminus, or both termini). For example, a fragment of a protein or polypeptide may comprise or consist of a 5-200, 5-150, 5-100, 5-50, 5-25, 5-15, 10-200, 10-150, 10-100, 10-50, 10-25, or 10-15 contiguous amino acid sequence of the full-length protein or polypeptide. An "immunogenic fragment" of a protein or polypeptide is a fragment of a protein or polypeptide typically at least 5 or 10 amino acids in length that includes one or more epitopes of the full-length protein or polypeptide (*e.g.,* a peptide present in the full-length protein or polypeptide).

Immune Stimulators and Vaccines. The compositions disclosed herein may include pharmaceutical compositions that are administered as immune stimulators or vaccines. Typically, the pharmaceutical composition comprises an effective amount or concentration of an immune stimulator and optionally an antigen for inducing a protective or therapeutic immune response against a disease, which may include, but is not limited to infection by a pathogen, cancer, or an autoimmune disease. Inducing a protective or therapeutic immune response may include inducing a Th1 response to one or more epitopes of a protein associated with the disease (*e.g.,* a protein associated with a pathogen, cancer, or autoimmune disease).

Where the disease relates to infection by a pathogen, inducing a protective response may include inducing sterilizing immunity against the pathogen. Inducing a therapeutic response may include reducing the pathogenic load of a subject, for example, as determined by measuring the amount of circulating pathogen before and after administering the composition. Inducing a therapeutic response may include reducing the degree or severity of at least one symptom of infection by the pathogen.

The presently disclosed methods may be utilized for inducing a protective or therapeutic immune response against disease by administering the pharmaceutical compositions disclosed herein (*e.g.,* as immunogenic compositions or vaccines) to a subject in need thereof, which may include a human or non-human having or at risk for acquiring the disease. The methods may include administering a first pharmaceutical composition and optionally may include administering a second pharmaceutical composition to augment or boost an immunogenic response induced by the first pharmaceutical composition. The first and second pharmaceutical compositions may be the same or different. The optionally administered second pharmaceutical composition may be administered prior to, concurrently with, or after administering the first pharmaceutical composition. In some embodiments, the first composition is administered and then the second composition is administered after waiting at least about 4, 5, or 6 weeks. The first composition (and the second composition) may be administered one or more times.

The presently disclosed compositions, kits, and methods may be utilized to protect against or treat infection by a pathogen. As used herein, a "pathogen" includes, but is not limited to a living microorganism such as bacteria, viruses, and fungi that cause disease in a host. Suitable pathogens for treatment of prevention by the compositions, kits, and methods disclosed herein may include pathogens that are susceptible to cell-mediated immune responses in the host (*e.g.,* Th1-mediated immune response) such as *Chlamydia* and infectious bursal disease virus (IBDV).

The presently disclosed compositions, kits, and methods also may be utilized to protect against or treat cancers or hyperproliferative disorders that are susceptible to cell-mediated immune responses in the host (*e.g.,* Th1-mediated immune response), which may include, but are not limited to adenocarcinoma, leukemia, lymphoma, melanoma, myeloma, sarcoma, and teratocarcinoma and particularly cancers of the adrenal gland, bladder, bone, bone marrow, brain, breast, cervix, gall bladder, ganglia, gastrointestinal tract, heart, kidney, liver, lung, muscle, ovary, pancreas, parathyroid, prostate, skin, testis, thymus, and uterus.

The presently disclosed compositions, kits, and methods also may be utilized to protect against or treat autoimmune diseases that are susceptible to cell-mediated immune responses in the host (*e.g.,* Th1-mediated immune response), which may include, but are not limited to autoimmune haematological disorders (including *e.g.* hemolytic anaemia, aplastic anaemia, pure red cell anaemia and idiopathic thrombocytopenia), systemic lupus erythematosus, polychondritis, sclerodoma, Wegener granulomatosis, chronic active hepatitis, myasthenia gravis, psoriasis, Steven-Johnson syndrome, idiopathic sprue, autoimmune inflammatory bowel disease (including *e.g.* ulcerative colitis, Crohn's disease and Irritable Bowel Syndrome), endocrine Graves disease, sarcoidosis, multiple sclerosis, primary biliary cirrhosis, juvenile diabetes (diabetes mellitus type I), uveitis (anterior and posterior), keratoconjunctivitis sicca and vernal keratoconjunctivitis, interstitial lung fibrosis, psoriatic arthritis or glomerulonephritis (with and without nephrotic syndrome, *e.g.* including idiopathic nephrotic syndrome or minimal change nephropathy).

The presently disclosed composition may be administered to potentiate or enhance an immune response. As used herein, "potentiating" or "enhancing" an immune response means increasing the magnitude and/or the breadth of the immune response. For example, the number of cells that recognize a particular epitope may be increased ("magnitude") and/or the numbers of epitopes that are recognized may be increased ("breadth"). Preferably, a 5-fold, or more preferably a 10-fold or greater, enhancement in T-cell responses may be obtained by administering the pharmaceutical composition disclosed herein.

The presently disclosed compositions, kits, and methods may be utilized to induce an immune response, including, but not limited to a cellular immune response such as a "Th1-response." As utilized herein, a Th1-response may be characterized by cytokine production such as interferons (*e.g.,* IFN-γ), tumor necrosis factor (*e.g.,* TNF-β), and interleukins (*e.g.,* IL-2). A Th1-response also may be characterized by increased killing efficiency of macrophages with respect to a pathogen and the proliferation of cytotoxic CD8⁺ cells against the pathogen. A Th1 response also may be characterized by the presence of opsonizing antibodies against the antigen. Th1-responses may be assessed as described previously. (*See* Li et al., Vaccine 28 (2010) 1598-1605, the content of which is incorporated herein by reference in its entirety).

In some embodiments, the presently disclosed compositions, kits, and methods may be utilized to induce a Th1-response preferentially relative to other responses, for example, a Th2-response. As utilized herein, a Th2-response may be characterized by cytokine production such as interleukins (*e.g.,* IL-4, IL-5, IL-6, IL-10, and IL-13). A Th2-response also may be characterized by B-cell stimulation and proliferation to induce B-cell antibody class switching and to increase neutralizing antibody production. Computer models have suggested that a Th1-response versus a Th2-response may be dependent on antigen dosage. (*See* Bergmann et al., Bulletin of Math. Biol. (2002) 64, 425-446; and Bergmann et al., Bulletin of Math. Biol. (2001) 63, 405-439, the contents of which are incorporated by reference in their entireties).

The presently disclosed composition, kits, and methods may be utilized to prevent or treat infections by pathogens that are susceptible to a T-cell mediated immune response (*e.g.,* a Th1 immune response). In some embodiments, the presently disclosed composition, kits, and methods may be utilized to prevent or treat infections by *Chlamydia* spp. As is the case for many other intracellular pathogens, T-lymphocytes play a key role in a protective host response to *Chlamydia* infection (Morrison et al., 1995, Infect. Immun. 63:4661-4668; Rank, 2006, In Chlamydia Genomics and Pathogenesis. P. M. Bavoil and B. Wyrick (ed.). Horizon Bioscience Press, Norfolk, U. K.). IFN-γ producing Th1 helper lymphocytes are indispensible for efficient and complete elimination of chlamydial infection (Perry et al., 1997, J. Immunol. 158:3344-3352; Rottenberg et al., 2000, J. Immunol. 164:4812-4818; Vuola et al., 2000, Infect. Immun. 68:960-964.), and ablation of Th1 cells or effector functions results in increased chlamydial disease and failure to eliminate chlamydiae (Cotter et al., 1997, Infect. Immun. 65:2145-2152; Lu et al., 2000, Mol. Med. 6:604-612; Morrison et al., 1995, Infect. Immun. 63:4661-4668; Wang et al., 1999, Eur. J. Immunol. 29:3782-3792.) They restrict chlamydial replication via Th1-type effector cytokines, most prominently IFN-γ, contributing to a DTH response (Perry *et al.,* 1997; Rottenberg *et al.,* 2000). Such protective DTH responses are characterized by tissue infiltration of CD4⁺ T cells and macrophages and release of proinflammatory Th1 cytokines such as IL-1, IL-2, IL-12, IFN-γ, or TNF-α.

### Formulation of the Pharmaceutical Compositions

The pharmaceutical compositions disclosed herein may be formulated as vaccines for administration to a subject in need thereof. Such compositions can be formulated and/or administered in dosages and by techniques well known to those skilled in the medical arts taking into consideration such factors as the age, sex, weight, and condition of the particular patient, and the route of administration.

The compositions may include pharmaceutical solutions comprising carriers, diluents, excipients (*e.g.,* powder excipients such as lactose, sucrose, and mannitol), and surfactants (*e.g.,* non-ionic surfactants such as Kolliphor HS 15, Kollidon 12 PF, and Tween-20), as known in the art. Further, the compositions may include preservatives (*e.g.,* anti-microbial or anti-bacterial agents such as benzalkonium chloride). The compositions also may include buffering agents (*e.g.,* in order to maintain the pH of the composition between 6.5 and 7.5).

The pharmaceutical compositions may be administered prophylactically or therapeutically. In prophylactic administration, the vaccines may be administered in an amount sufficient to induce a cellular immune response for protecting against infection or for treating infection. In therapeutic applications, the vaccines are administered to a patient in an amount sufficient to elicit a therapeutic effect (*e.g.,* an immune response to the administered antigen, which cures or at least partially arrests or slows symptoms and/or complications of disease (*i.e.,* a "therapeutically effective dose")).

The compositions disclosed herein may be delivered via a variety of routes. Typical delivery routes include parenteral administration (*e.g.,* intradermal, intramuscular, intraperitoneal, or subcutaneous delivery). Other routes include intranasal and intrapulmonary routes. Further routes include oral administration, intravaginal, and intrarectal routes. Formulations of the pharmaceutical compositions may include liquids (*e.g.,* solutions and emulsions), sprays, and aerosols. In particular, the compositions may be formulated as aerosols or sprays for intranasal or intrapulmonary delivery. Suitable devices for administering aerosols or sprays for intranasal or intrapulmonary delivery may include inhalers and nebulizers.

The compositions disclosed herein may be co-administered or sequentially administered with other immunological, antigenic or vaccine or therapeutic compositions, including an adjuvant, or a chemical or biological agent given in combination with an antigen to enhance immunogenicity of the antigen. Additional therapeutic agents may include, but are not limited to, cytokines such as interferons (*e.g.,* IFN-γ) and interleukins (*e.g.,* IL-2).

### Prime-Boost Vaccination Regimen

As used herein, a "prime-boost vaccination regimen" refers to a regimen in which a subject is administered a first composition and then after a determined period of time (*e.g.,* after about 2, 3, 4, 5, or 6 weeks), the subject is administered a second composition, which may be the same or different than the first composition. The first composition (and the second composition) may be administered one or more times. The disclosed methods may include priming a subject with a first composition by administering the first composition at least one time, allowing a predetermined length of time to pass (*e.g.,* at least about 2, 3, 4, 5, or 6 weeks), and then boosting by administering the same composition or a second, different composition.

### Characterization of the Immune Response in Vaccinated Individuals

The pharmaceutical compositions disclosed herein may be delivered to subjects at risk for a disease (*e.g.,* infection with a pathogen) or to subjects who have acquired the disease (*e.g.,* subject who are infected with a pathogen). In order to assess the efficacy of an administered immunogenic composition or vaccine, the immune response can be assessed by measuring the induction of cell-mediated responses and/or antibodies to particular epitopes. T-cell responses may be measured, for example, by using tetramer staining of fresh or cultured PBMC, ELISPOT assays or by using functional cytotoxicity assays, which are well-known to those of skill in the art. Antibody responses may be measured by assays known in the art such as ELISA. Titer or load of a pathogen may be measured using methods in the art including methods that detect nucleic acid of the pathogen. (*See, e.g.,* U.S. Patent No. 7,252,937, the content of which is incorporated by reference in its entirety). Immune responses also may be characterized by physiological responses. (*See* Li et al., Vaccine 28 (2010) 1598-1605; and Stemke-Hale et al., Vaccine 2005 Apr 27;23(23):3016-25, the content of which re incorporated herein by reference in their entireties.) Immune response also may be measured by pathological responses such as total weight loss or gain for the animal (*e.g.,* weight loss associated with infection by a pathogen). Immune response also may be measured by pathological responses such as weight loss or gain for an organ of the animal (*e.g.,* lung-weight gain in mice infected with *C. abortus,* or weight loss or gain for the Bursa of Fabricius in chicken infected with infectious bursa disease virus).

The invention is further defined in the following numbered clauses:
1. A method for stimulating an immune response in a subject in need thereof, the method comprising administering a composition comprising biodegradable particles, the biodegradable particles having an effective average diameter of 0.5-10 um.
2. The method of clause 1, wherein the biodegradable particles comprise polymeric material.
3. The method of clause 2, wherein the polymeric material comprises carbohydrate monomers.
4. The method of clause 1, wherein the composition further comprises a powder excipient for the biodegradable particles.
5. The method of clause 1, wherein the biodegradable particles are formed by a process that includes spray-drying.
6. The method of clause 1, wherein the composition comprises a suspension of the biodegradable particles.
7. The method of clause 1 comprising administering the biodegradable particles to the subject at dose between (BW/20)^{3/4} µg and 100 x ((BW/20)^{3/4}) µg, wherein BW is the body weight of the subject in grams.
8. The method of clause 1, wherein the composition further comprises an adjuvant.
9. The method of clause 1, wherein the composition further comprises an apoptosis inhibitor.
10. The method of clause 1, wherein the composition further comprises an antigen.
11. The method of clause 10, wherein the biodegradable particles and the antigen are present in the composition at a concentration ratio of 0.00018 - 18.0 fmole antigen/µg biodegradable particles.
12. The method of clause 10 comprising administering the antigen at a dose level of 0.2-2.0 pg antigen/g body weight of the subject.
13. The method of clause 1, wherein the composition further comprises an apoptosis inhibitor and an antigen and the method comprises administering the antigen at a dose level of less than 0.2 pg antigen/g body weight of the subject.
14. The method of clause 1, wherein the method consists of administering a composition consisting essentially of a suspension of the particles.
15. The method of clause 1, wherein the method consists of administering a composition consisting essentially of a suspension of the particles in a solution of a non-ionic surfactant and an adjuvant.
16. The method of clause 1, wherein the method consists of administering a composition consisting essentially of a suspension of the particles in a solution of a non-ionic surfactant, an adjuvant, and an apoptosis inhibitor.
17. The method of clause 1, wherein the method consists of administering a composition consisting essentially of a suspension of the particles in a solution of a non-ionic surfactant, an adjuvant, an apoptosis inhibitor, and one or more peptide antigens.
18. The method of clause 1, wherein the method stimulates T cell immunity against infection by a pathogen.
19. The method of clause 1, wherein the method stimulates a Th1 cell immune response.
20. The method of clause 1, wherein the method stimulates an immune response in the subject that causes the subject to gain weight at a higher relative rate than a subject that has not been administered the composition.
21. The method of clause 20, wherein the subject is a fowl.
22. A composition comprising:
   (a) biodegradable particles, the biodegradable particles having an effective average diameter of 0.5-10 um;
   (b) an adjuvant; and
   (c) an apoptosis inhibitor.
23. The composition of clause 22, further comprising:
   (d) an antigen.
24. A composition consisting of:
   (a) biodegradable particles, the biodegradable particles having an effective average diameter of 0.5-10 um;
   (b) an adjuvant; and
   (c) an apoptosis inhibitor.

### EXAMPLES

The following examples are illustrative and are not intended to limit the disclosed subject matter.

### Glossary of Terms and Acronyms and Description of Experiments

PLGA RG502H: biodegradable poly (lactide-co-glycolide-poly (50%:50%) copolymer.
PLGA-PEG: biodegradable poly (lactide-co-glycolide-poly (50%:50%) copolymer, chemically modified by incorporation of 5% polyethylene glycol (PEGylation) with a molecular weight of 5,000.
PLA L206S: biodegradable poly(L-lactide) polymer.
Nelfinavir: nelfinavir mesylate, which is an inhibitor of the protease of human immunodeficiency virus and is used as an antiretroviral drug. Nelfanivir also has immunostimulatory and/or immunopotentiating activity and is poorly water soluble.
Pluronic L121: polyoxyethylene-polyoxypropylene block copolymer, which is a surfactant and an adjuvant.
Resiquimod or R-848: selective ligand for the Toll-like receptor 7 (TLR7) in the mouse, which is an adjuvant.
Trehalose-di-behenate: synthetic analogue of trehalose dimycolate from the cord factor of the tubercle bacillus, which is an adjuvant.
Poly (I:C): double-stranded polyribonucleotide poly(I)·poly(C), which is an adjuvant and which induces interferon.
Q-VD-OPH: pan-caspase inhibitor that irreversibly binds to activated caspases to block apoptosis. Q-VD-OPH is composed of the amino acids valine and aspartate with N-terminal quinolyl and a C-terminal di-fluorophenoxy methylketone group. Q-VD-OPH is highly cell permeable and non-toxic *in vitro* and *in vivo* at high concentrations.
Emricasan or IDN-6556: broad-spectrum irreversible caspase inhibitor that blocks apoptosis.
Kolliphor HS 15: polyethylene glycol-15-hydroxy stearate, USP: Polyoxyl-15-Hydroxystearate, Ph. Eur.: Macrogol 15 Hydroxystearate, which is a non-ionic solubilizer and emulsifying agent.
Kollidon 12 PF: soluble polyvinylpyrrolidone with a molecular weight of 2,000-3,000, which is a solubilizing agent, dispersant, and crystallization inhibitor.
Tween-20: non-ionic polyoxyethylene surfactant.
Benzalkonium Chloride: quaternary ammonium compound with antimicrobial efficacy over a wide pH range at a concentration of less than 0.02%.
PBS: phosphate-buffered saline.
IBDV: Infectious Bursal Disease Virus, which is double-stranded DNA virus of the *Birnavirus* family causes an infection of the Bursa of Fabricius in young chickens which destroys the B lymphocytes in this organ. The absence of B lymphocytes renders these chickens unable to produce antibodies and makes them therefore highly susceptible to many infectious diseases.
C3H/HeJ: inbred mouse strain that has a mutation in the Toll-like receptor 4 *(TLR4)* gene that results in a truncated (shortened) TLR4 protein. TLR-4 binds bacterial lipopolysaccharide (LPS) and triggers the inflammatory response after LPS exposure. Due to the virtually absent LPS response, C3H/HeJ mice mount very little innate inflammatory response and also exhibit an aberrant adaptive immune response. These mice are extremely sensitive to gram-negative bacteria including chlamydiae. After chlamydial intranasal challenge infection they remain without discernible clinical symptoms for about one week, but then a large fraction of challenged mice die over the next two weeks due to uninhibited overwhelming growth of chlamydiae. Treatment with immune stimulators can partially substitute for the absent TLR4 signaling and make these mice more resistant to chlamydial infection. Therefore, immune stimulator experiments in mice were conducted with this mouse strain.
A/J: inbred mouse strain that produces a weak innate immune response with low inflammation. After intranasal challenge with chlamydiae, naive mice of this strain develop severe disease and high chlamydial lung loads due to poor control of chlamydial growth. In contrast, mice that are immune to chlamydiae due to previous exposure to a low level infection or due to successful vaccination are fully protected and do not develop disease. Therefore, vaccine experiments were in part conducted with this mouse strain.
129S6: inbred mouse strain that has a defect in the effector arm of the innate immune system with aberrant DAP12 signaling in natural killer (NK) T cells. Due to this defect, naive 129S6 mount a weak innate immune response to chlamydiae and are highly susceptible to *C. abortus.* In contrast, 129S6 mice mount a fully protective adaptive immune response after exposure to a low level infection or due to successful vaccination, and are fully protected and do not develop disease after vaccination. Therefore, vaccine experiments were in part conducted with this mouse strain.
Mouse experiments: In the mouse challenge for infection experiments with *Chlamydia abortus,* only female mice are used because they can be maintained in cages without the severe fighting that is common for male mice. Typically, for immune stimulator experiments without prior vaccination, mice of 10 weeks of age are used. For vaccine experiments, younger mice of about 6 weeks of age are first vaccinated or receive an immunizing low-dose intranasal *C. abortus* infection, and are challenged with a high dose infection 4-6 weeks later at 10-12 weeks of age. For intranasal challenge, the mice receive a light isoflurane anesthesia, and 20 µl of a suspension of 10⁸ to 3×10⁸ *C. abortus* viable bacteria in sucrose-phosphate-glutamate buffer are deposited on the nostrils, from where the mice inhale the bacteria. For immune stimulator experiments, the mice are observed until 3 weeks later and surviving mice are then euthanized. In vaccine experiments, mice are euthanized between 10-12 days after challenge.
   In the vaccine experiments, mouse lungs were weighed and homogenized, DNA was extracted, and *C. abortus* genome lung loads are determined by a real-time PCR targeting the *Chlamydia* spp. 23S rRNA gene. All mouse experiments are approved by the Auburn University Institutional Animal Care and Use Committee (IACUC).
   In all vaccine experiments, the antigens used were 20-mer peptides of the C. *abortus* vaccine candidate proteins Dnax2, GatA, GatC, Omp90A, Pbp3. These peptides overlapped by 10 amino acids and therfore comprised all possible 10-mer peptides of these proteins.
Chicken experiments: In the chicken experiments for evaluation of the immune stimulator treatment on resistance to naturally circulating infections and/or on body growth without experimental challenge infection, freshly hatched standard hybrid broiler chickens weighing 45-50 g were used on the day of hatching and subcutaneously injected with the microparticle immune stimulator. The body weight of these chickens was determined after 3 weeks.
   For IBDV vaccine experiments, freshly hatched standard hybrid broiler chickens were immunized with microparticles containing 20-mer overlapping peptides of all proteins of the IBDV virus. After 3 weeks, the chickens received an intranasal challenge of the virus suspended in PBS. The chickens were weighed and euthanized after 7 days, and sex, and weight and appearance of the Bursa of Fabricius were determined.

### Example 1. Immune Stimulator Experiment in Mice

The ability of a composition comprising a suspension of biodegradable particles to induce an innate immune response as an "immune stimulator" in mice was tested under the parameters of Table 1.

**Table 1**

| | |
|---|---|
| Model System | Immune Stimulator Experiment in Mice: day-21 post-challenge termination survival analysis |
| Mouse strain | C3H/HeJ, 10 weeks old at start of experiment |
| Challenge | 10⁸ *C*. *abortus* elementary bodies, 21, 11, or 1 days after immune stimulator |
| Administration | 1× intranasal (IN) 10 µg microparticle immune stimulator preparation & 30 µg lactose microfine powder in 20 µl PBS/0.1% Kolliphor HS 15 1x PBS control: 20 µl intranasal |
| Formulation | Microparticles (1-10 µm) spray-dried from PLGA-PEG & Pluronic L121 (3:2) |
| Conclusion | Local (intranasal) administration of the microparticle immune stimulator at the site of the challenge inoculation was found to be effective given 1 day before challenge up to at least 3 weeks before the challenge inoculation. |

As indicated in Table 1, microparticles having an effective average diameter of 1-10 µm were prepared by spray-drying a solution of PLGA-PEG and Pluronic L121 (3:2). (*See* Figure 1A). In order to prepare the immune stimulator, 10 µg of the microparticles were mixed with 30 µg lactose microfine powder and added to 20 µl PBS/0.1% Kolliphor HS 15 to form a suspension. The immune stimulator (20 µl) was administered intranasally to 10 week old mice (C3H/HeJ strain). As a control, IX PBS was administered. The mice were challenged at 1, 11, or 21 days post-administration by administering intranasally 10⁸ *C*. *abortus* elementary bodies. The results presented in Figure 1B, C, and D illustrate that mice administered the immune stimulator exhibited a higher survival rate than mice administered the control. These results demonstrate that an immune stimulator that did contain an antigen against *C*. *abortus* may be administered in order to induce innate immunity.

### Example 2. Immune Stimulator Experiment in Mice

The ability of a composition comprising a suspension of biodegradable particles and an adjuvant to induce an innate immune response as an "immune stimulator" in mice was tested under the parameters of Table 2.

**Table 2**

| | |
|---|---|
| Model System | Immune Stimulator Experiment in Mice: day-21 post-challenge termination survival analysis |
| Mouse strain | C3H/HeJ, 10 weeks old at start of experiment |
| Challenge | 3×10⁸ *C. abortus* elementary bodies, 1 day after immune stimulator |
| Administration | 1× intranasal, 10 µg of each microparticle immune stimulator preparation suspended in 20 µl suspension buffer (0.1% Kolliphor HS 15, 0.001 % Benzalkonium Chloride in PBS); prior to suspension, each immune stimulator preparation was mixed with the 3-fold amount of lactose microfine powder. |
| Formulation | Microparticles (0.5-10 µm) spray-dried from a biodegradable carrier & adjuvant: |
| | PLGA RG502H and adjuvants: |
| | 1) Pluronic L121: 3.5 µg Pluronic L121 and 6.5 µg PLGA RG502H; |
| | 2) Resiquimod: 0.2 µg resiquimod and 9.8 µg PLGA RG502H; |
| | 3) No adjuvant: 10 µg PLGA RG502H. |
| Conclusions | 1) A PLGA RG502H plus adjuvant microparticle preparation administered intranasally at 10 µg per mouse was an effective immune stimulator. |
| | 2) Addition of adjuvants to the PLGA RG502H microparticles augmented the immune stimulatory effect. |
| | 3) Different adjuvants mediated a similar immune stimulatory effect. |

As indicated in Table 2, microparticles having an effective average diameter of 0.5-10 µm were prepared by spray-drying a solution of PLGA RG502H (poly (lactide-co-glycolide-poly (50%:50%) copolymer) and adjuvants. Microparticle formulation 1) included 6.5 µg PLGA RG502H and 3.5 µg Pluronic L121; Microparticle formulation 2) included 9.8 µg PLGA RG502H and 0.2 µg resiquimod; and Microparticle formulation 3) included 10 µg PLGA RG502H (*i.e.* no adjuvant). The microparticle formulations were mixed a 3-fold amount of lactose microfine powder and added to 20 µl suspension buffer (0.1% Kolliphor HS 15, 0.001 % Benzalkonium Chloride in PBS) to prepare an immune stimulator. The immune stimulator (20 µl) was administered intranasally to 10 week old mice (C3H/HeJ strain). The mice were challenged at 1 day post-administration by administering intranasally 10⁸ *C. abortus* elementary bodies. The results presented in Figure 2A and B illustrate that mice administered the immune stimulator comprising an adjuvant exhibited a higher survival rate and a lower body weight loss than mice administered the immune stimulator lacking an adjuvant. These results demonstrate that adjuvants may used to augment the effect of an immune stimulator comprising the microparticles to induce innate immunity.

### Example 3. Immune Stimulator Trial in Chickens

The ability of a composition comprising a suspension of biodegradable particles to induce an innate immune response as an "immune stimulator" in chickens was tested under the parameters of Table 3.

**Table 3**

| | |
|---|---|
| Model System | Immune Stimulator Trial in Chickens: Two pooled growth experiments from hatching to day 28, with immune |
| | stimulator or control suspension buffer administration on the day of hatching; survival analysis of impact on losses of chickens |
| Chicken strain | Standard broiler chickens |
| Treatment | 1× 200 µl subcutaneously |
| Challenge | NONE |
| Formulations | 1) 270 µg Microparticle Immune Stimulator (1-10 µm) spray-dried from PLGA-PEG & Pluronic L121 (6.5:3.5) combined with & 810 µg lactose microfine powder |
| | 2) Suspension Buffer (200 µl: 0.1% Kolliphor HS 15, 0.001 % Benzalkonium Chloride in PBS) |
| Dosage | The microparticle immune stimulator dose was allometrically scaled 27-fold for 1,600 g target weight of chickens at 3 weeks as compared to 10 µg microparticle immune stimulator for 20 g mouse target weight: (1,600/20)^{3/4} = 26.7. |
| Antigen | NONE |
| Conclusion | Subcutaneous administration of microparticle immune stimulator delivered as spray-dried PLGA-PEG microparticles that contain co-polymer adjuvant was effective. The effect was observed using a single 270 microgram total dose of the immune stimulator. |

As indicated in Table 3, microparticles having an effective average diameter of 1-10 µm were prepared by spray-drying a solution of PLGA-PEG and Pluronic L121 (6.5:3.5). In order to prepare the immune stimulator composition, 270 µg of the microparticles were combined with 810 µg lactose microfine powder and added to 200 µl of suspension buffer (0.1% Kolliphor HS 15, 0.001 % Benzalkonium Chloride in PBS). The 270 µg of the microparticles was arrived at by allometrically scaling the 10 µg microparticle amount used for a 20 g mouse based on the formula (BW/20)3/4 = allometric scaling factor, where "BW" equals the body weight of the target animal in grams. Assuming that the target weight of a chicken is 1600 g, the allometric scaling factor is (1,600/20)^{3/4} = 26.7. Multiplying the 10 µg microparticle amount used for a 20 g mouse by the scaling factor of 26.7 results in ∼270 µg.

The immune stimulator thus formulated (200 µl) was administered subcutaneously to standard broiler chickens on the day of hatching. Suspension buffer without microparticles was administered as a control. The chickens were not challenged and the cumulative survival rate was determined for chickens administered the immune stimulator versus control. The results presented in Figure 3 illustrate that chickens administered the immune stimulator exhibited a higher cumulative survival rate than chickens administered the control. These results demonstrate that an immune stimulator that did contain an antigen may be administered in order to increase survival rate in chickens.

### Example 4. Immune Stimulator and Growth Enhancement Trial in Chickens

The ability of a composition comprising a suspension of biodegradable particles to induce an innate immune response as an "immune stimulator" and to enhance growth in chickens was tested under the parameters of Table 4.

**Table 4**

| | |
|---|---|
| Model System | Immune Stimulator & Growth Enhancement Trial in Chickens: Evaluation of immune stimulator effect on body weight |
| Animal | chickens, hatched on starting day of experiment |
| Administration | 1× subcutaneous 270 µg microparticle immune stimulator preparation & 810 µg lactose microfine powder in 200 µl PBS/0.1% Kolliphor HS 15 1x PBS control: 200 µl subcutaneous |
| Formulation | Microparticles (1-10 µm) spray-dried from PLGA-PEG & Pluronic L121 (6.5:3.5) |
| Conclusions | Subcutaneous injection of allometrically scaled 270 µg of the microparticle immune stimulator on the day of hatching increases the body weight of 22-day old chickens by 116 g from 886 g to 1012 g (14.2%). |

As indicated in Table 4, microparticles having an effective average diameter of 1-10 µm were prepared by spray-drying a solution of PLGA-PEG and Pluronic L121 (6.5:3.5). In order to prepare the immune stimulator composition, 270 µg of the microparticles were combined with 810 µg lactose microfine powder and added to 200 µl of suspension buffer (0.1% Kolliphor HS 15, 0.001 % Benzalkonium Chloride in PBS).

The immune stimulator thus formulated (200 µl) was administered subcutaneously to standard broiler chickens on the day of hatching. Suspension buffer without microparticles was administered as a control. The chickens were not challenged and weight gain was assessed 21 days post-administration. The results presented in Figure 4 illustrate that chickens administered the immune stimulator exhibited a higher average body weight than chickens administered the control. These results demonstrate that an immune stimulator that did contain an antigen may be administered in order to enhance growth in chickens.

### Example 5.

The ability of a composition comprising a suspension of biodegradable particles to induce an innate immune response as an "immune stimulator" and to improve feed conversion rate in chickens was tested under the parameters of Table 5.

**Table 5**

| | |
|---|---|
| Model System | Immune Stimulator & Growth Enhancement Trial in Chickens: Evaluation of immune stimulator effect on feed conversion |
| Chicken strain | Standard broiler chickens |
| Challenge | NONE |
| Treatment | 1× subcutaneous 270 µg microparticle immune stimulator preparation & 810 µg lactose microfine powder in 200 µl suspension buffer (PBS/0.1% Kolliphor HS 15/0.001 % Benzalkonium Chloride) at hatching 1x control: 200 µl suspension buffer subcutaneously at hatching |
| Formulation | Microparticles (1-10 µm) spray-dried from PLGA-PEG & Pluronic L121 (6.5:3.5) |
| Antigen Dose | NONE |
| Conclusion | A single subcutaneous, allometrically scaled dose of the microparticle immune stimulator significantly reduced the feed conversion rate from 1.264 g feed per 1.0 g body weight gain in control chickens to 1.243 g feed per 1.0 g body weight gain in immune stimulator-treated chickens. |

As indicated in Table 5, microparticles having an effective average diameter of 1-10 µm were prepared by spray-drying a solution of PLGA-PEG and Pluronic L121 (6.5:3.5). In order to prepare the immune stimulator composition, 270 µg of the microparticles were combined with 810 µg lactose microfine powder and added to 200 µl of suspension buffer (0.1% Kolliphor HS 15, 0.001 % Benzalkonium Chloride in PBS).

The immune stimulator thus formulated (200 µl) was administered subcutaneously to standard broiler chickens on the day of hatching. Suspension buffer without microparticles was administered as a control. The sample size was 96 female broiler chickens each in 12 battery pens of 8 chickens fed for 3 weeks. The chickens were not challenged and feed conversion rate was assessed by measuring feed consumption per body weight (*i.e.,* g feed required per body weight gain). The results presented in Figure 5 illustrate that chickens administered the immune stimulator exhibited a better feed conversion rate than chickens administered the control. These results demonstrate that an immune stimulator that did contain an antigen may be administered in order to improve feed conversion rate.

### Example 6. Immune Stimulator Trial in Mice with Apoptosis Inhibitor

The ability of a composition comprising a suspension of biodegradable particles and an apoptosis inhibitor to induce an innate immune response as an "immune stimulator" and to enhance survival rate in mice was tested under the parameters of Table 6.

**Table 6**

| | |
|---|---|
| Model System | Immune Stimulator Trial in Mice: day-21 post-challenge termination survival analysis |
| Mouse strain | C3H/HeJ, 10 weeks old at start of experiment |
| Challenge | 10⁸ *C*. *abortus* elementary bodies 3 days after treatment |
| Treatment | 1× intraperitoneally (in 200 µl HBSS) & 1/10 dose intranasal (in 20 µl HBSS) |
| Carriers | 1) 8 µg nelfinavir & 3.6 µg Pluronic L121® |
| | 2) 8 µg nelfinavir & 3.6 µg Pluronic L121® + 0.16 µg Q-VD-OPH (apoptosis- inhibitor) |
| Immune Stimulator Dose | Immune stimulator delivered as a total of 12 µg composed of ∼5 µm spray-dried microparticles |
| Conclusion | Inhibition of apoptosis modulates the innate immune response from non-protective to protective if a small-molecule inhibitor of apoptosis is included in an immune stimulator delivered as spray-dried nelfinavir microparticles that contain co-polymer adjuvant. This effect occurs at a single -12 microgram total dose of the immune stimulator. |

As indicated in Table 6, microparticles were prepared from nelfanivir (8 µg) combined with Pluronic L121® (3.6 µg) and added to HBSS (200 µl) to prepare an immune stimulator. The apoptosis inhibitor Q-VD-OPH was added (0.16 µg) to test its effect on immune stimulation. The immune stimulator was administered intraperitoneally (200 µl) and intranasally (20 µl) to C3H/HeJ which were 10 weeks old at the start of the experiment. The mice then were challenged at 3 days post-administration by administering intranasally 10⁸ *C. abortus* elementary bodies. The results presented in Figure 6 illustrate that mice administered the immune stimulator comprising an apoptosis inhibitor exhibited a higher cumulative survival rate than mice administered the immune stimulator lacking an apoptosis inhibitor. These results demonstrate that apoptosis inhibitors may used to augment the effect of an immune stimulator comprising the microparticles to induce innate immunity.

### Example 7. Vaccine Trial in Mice with Apoptosis Inhibitor and Antigen

The ability of a composition comprising a suspension of biodegradable particles, an apoptosis inhibitor (Q-VD-OPH or emricasan), and an antigen to induce a protective T cell response versus a non-protective/pathogenic response in mice was tested under the parameters of Table 7.

**Table 7**

| | |
|---|---|
| Model System | Vaccine Trial in Mice |
| | *C. abortus* respiratory challenge model, termination day-11 post-challenge. Body weight change and lung weight were analyzed on day-11 post-challenge. |
| Mouse strain | 129S6, 6 weeks old at treatment |
| Challenge | 3×10⁸ *C. abortus* elementary bodies 6 week after treatment |
| Treatment | 1× intranasal in 20 µl suspension buffer |
| Carriers/ Controls | 1. Vaccine: 6.5 µg PLGA-PEG & 3.6 µg Pluronic L121® + *C. abortus* peptides |
| | 2. Vaccine + Apoptosis Inhibitor: 6.5 µg PLGA-PEG & 3.6 µg Pluronic L121® + *C*. *abortus* peptides + 0.2 µg Q-VD-OPH |
| | 3. Vaccine + Apoptosis Inhibitor: 6.5 µg PLGA-PEG & 3.6 µg Pluronic L121® + *C*. *abortus* peptides + 0.2 µg emricasan |
| | 4. Vaccine Carrier: 6.5 µg PLGA-PEG & 3.6 µg Pluronic L121® |
| | 5. Live Vaccine: low-dose C. *abortus* intranasal inoculation (mediates maximum protection) |
| Antigen/ Vaccine Dose | 0.2 femtoMoles of each overlapping 20-mer peptide from the 5 best protective *C. abortus* proteins in a total of 10 µg vaccine |
| Conclusion | Inhibition of apoptosis modulates the vaccine T cell immune response of mice from non-protective/pathogenic to protective if a small-molecule inhibitor of apoptosis is included in a vaccine delivered as spray-dried PLGA-PEG microparticles that contain co-polymer adjuvant. This effect occurs at a single ∼10 microgram total dose of the vaccine. |

As indicated in Table 7, microparticles were prepared from PLGA-PEG (6.5 µg) and added to Pluronic L121 (3.6 µg) to form a microparticle composition for use as a carrier and control. (*See* Vaccine Carrier 4. in Table 7). A vaccine was prepared by adding *C. abortus* peptides to the carrier in the form of 0.2 femtoMoles of each overlapping 20-mer peptide from 5 protective *C. abortus* proteins (see U.S. Published Application No. 2012/0009220, the contents of which are incorporated herein by reference in their entirety) in a total of 10 µg vaccine. *(See* Vaccine 1. in Table 7). A vaccine composition comprising an apoptosis inhibitor also was prepared by adding 0.2 µg Q-VD-OPH (see Vaccine + Apoptosis Inhibitor 2. in Table 7) or 0.2 µg emricasan (see Vaccine + Apoptosis Inhibitor 3., Table 7). A live vaccine was utilized as a control. (*See* Live Vaccine 5. in Table 7). The vaccine compositions and controls were administered intranasally (20 µl) to 6 week old mice (strain 129S6). The mice were challenged at 6 weeks post-administration by administering intranasally 10⁸ *C. abortus* elementary bodies. The results presented in Figure 7A, B, C, and D illustrate that mice administered the vaccine containing the apoptosis inhibitor exhibited the lowest body weight loss and lowest lung weight gain similar to the live vaccine, suggesting that the apoptosis inhibitor modulated the T cell response from non-protective/pathogenic to protective.

### Example 8. Vaccine Trial in Chickens with Apoptosis Inhibitor and Antigen

The ability of a composition comprising a suspension of biodegradable particles, an apoptosis inhibitor, and an antigen to induce a protective T cell response versus a non-protective/pathogenic response in chickens was tested under the parameters of Table 8.

**Table 8**

| | |
|---|---|
| Model System | Vaccine Trial in Chickens |
| | Infectious bursal disease virus (IBDV) respiratory challenge model, termination day-7 post-challenge. |
| Chickens | Standard broiler chickens, treatment on day of hatching |
| Challenge | IBDV suspension intranasal on 3 weeks after treatment |
| Treatment | 1× subcutaneous in 200 µl suspension buffer |
| Carriers/ Controls | 1. Vaccine: 175.5 µg PLGA-PEG & 94.5 µg Pluronic L121® + IBDV peptides |
| | 2. Vaccine + Apoptosis Inhibitor: 175.5 µg PLGA-PEG & 94.5 µg Pluronic L121® + IBDV peptides + 2.7, 5.4, or 10.8 µg Q-VD-OPH |
| | 3. Suspension buffer-treated chickens and IBDV challenge |
| | 4. Suspension buffer-treated chickens and no IBDV challenge (no disease) |
| Antigen/ Vaccine Dose | 0.54 femtoMoles of each overlapping 20-mer peptide from all IBDV virus proteins in a total of 270 µg microparticle vaccine, allometrically scaled 27-fold for 1,600 g target weight of chickens at 3 weeks as compared to 10 µg microparticle vaccine for 20 g mouse target weight: (1,600/20)^{3/4} = 26.7. |
| Conclusion | Inhibition of apoptosis modulates the vaccine T cell immune response of chickens from non-protective/pathogenic to protective if a small-molecule inhibitor of apoptosis is enclosed in a vaccine delivered as spray-dried PLGA-PEG microparticles that contain co-polymer adjuvant. This effect occurs at a single ∼270 microgram total dose of the vaccine. |

As indicated in Table 8, microparticles prepared from a solution of PLGA-PEG and Pluronic L121 (175.5 µg: 94.5 µg) and IBDV peptides consisting of 0.54 femtoMoles of overlapping 20-mer peptides from all IBDV virus proteins. Apoptosis inhibitor Q-VD-OPH was included in amounts of 2.7, 5.4, or 10.8 µg. In order to prepare the vaccine compositions, the microparticles were added to 200 µl of suspension buffer.

The vaccine compositions and control were administered subcutaneously to standard broiler chickens on the day of hatching. Suspension buffer alone was administered as a control. The chickens were challenged three weeks post-administration by administering IBDV. The weight and inflammation of Bursa of Fabricius were analyzed on day-7 post-challenge, where IBDV selectively targets B cells in the Bursa of Fabricius of chickens, leading to inflammation followed by atrophy. This reduces the weight of the Bursa after the inflammation has subsided. Inflammation was scored on a scale of 1-4, and the increase of bursa weight caused by inflammation was corrected by dividing bursa weight with the inflammation score. The results presented in Figure 8A, B, C, D, E, F, G, and H suggest that the apoptosis inhibitor modulated the T cell response from non-protective/pathogenic to protective in immunized chickens challenged with IBDV.

### Example 9. Vaccine Experiment in Mice

The ability of a composition comprising a suspension of biodegradable particles comprising nelfinavir and an antigen to induce a protective T cell response in mice was tested under the parameters of Table 9.

**Table 9. Vaccine Experiment in Mice**

| | |
|---|---|
| Model System | Vaccine Experiment in Mice: day-21 post-challenge termination survival analysis |
| Mouse strain | C3H/HeJ, 6 weeks old at start of experiment |
| Challenge | 10⁸ *C*. *abortus* elementary bodies 4 weeks after 3^{rd} vaccination |
| Vaccination | 3× in 4-week interval subcutaneously between shoulders |
| Antigen Dose | 0.1 femtoMole each peptide (0.22 pg each peptide) per mouse |
| Vaccine Carrier | 2 mg nelfinavir & 50 µg Poly (I:C) in 200 µl HBSS= antigen delivery as 1-20 µm microparticles of precipitated and grinded nelfinavir with co-precipitated Poly (I:C) adjuvant and peptide antigens |
| Conclusion | Effective low-antigen dose vaccination can utilize different adjuvants and different microparticle delivery modalities. |

As indicated in Table 9, microparticles having an effective diameter of (1-20 µm) were prepared by grinding nelfinavir, which is a very poorly water soluble HIV protease inhibitor with that has an immunopotentiating effect. The ground nelfinavir particles were co-precipitated with 50 µg Poly (I:C) and peptide antigens in the form of 0.1 femtoMoles of each overlapping 20-mer peptide from 5 *C. abortus* proteins shown to be protective against infection (see U.S. Published Application No. 2012/0009220, the contents of which are incorporated herein by reference in their entirety). The co-precipitated particles were added to 200 µl HBSS to form a vaccine composition. The vaccine compositions and control (200 µl HBSS) were administered subcutaneously between the shoulders of 6 week old mice (strain C3H/HeJ) for three times in four week intervals post-vaccination. The mice were challenged at 4 weeks after the third vaccination by administering intranasally 10⁸ *C. abortus* elementary bodies and the cumulative proportion of surviving mice was assessed. As indicated in Figure 9, vaccinated mice exhibited -70% cumulative survival while control mice exhibited -10% cumulative survival.

### Example 10. Vaccine Experiment in Mice

The ability of a composition comprising a suspension of biodegradable particles comprising nelfinavir and an antigen to induce a protective T cell response in mice was tested under the parameters of Table 10.

| | |
|---|---|
| Model System | Vaccine Experiment: day-21 post-challenge termination survival analysis |
| Mouse strain | C3H/HeJ, 6 weeks old at start of experiment |
| Challenge | 10⁸ *C*. *abortus* elementary bodies 6 weeks after vaccine |
| Vaccination | 1× subcutaneously between shoulders |
| Antigen Dose | 1 femtoMole each peptide (2.2 pg each peptide) per mouse |
| Vaccine Carrier | 5 mg nelfinavir & 0.25 mg Pluronic L121® in 200 µl HBSS= antigen delivery as 1-10 µm spray-dried microparticles |
| Conclusion | Effective low-antigen dose vaccination by microparticle delivery can utilize single vaccination of spray-dried preparations using nelfinavir as carrier and co-polymer as adjuvant. |

As indicated in Table 10, microparticles having an effective diameter of (1-10 µm) were prepared by spray-drying nelfinavir (5 mg) and Pluronic L121® (0.25 mg). The microparticles were administered with peptide antigens in the form of 1.0 femtoMoles of each overlapping 20-mer peptide from 5 *C. abortus* proteins shown to be protective against infection (see U.S. Published Application No. 2012/0009220, the contents of which are incorporated herein by reference in their entirety) in 200 µl HBSS. The vaccine compositions and control (200 µl HBSS) were administered subcutaneously between the shoulders of 6 week old mice (strain C3H/HeJ). The mice were challenged at 6 weeks post-vaccination by administering intranasally 10⁸ *C. abortus* elementary bodies and the cumulative proportion of surviving mice was assessed. As indicated in Figure 10, vaccinated mice exhibited -60% cumulative survival while un-treated mice exhibited -20% cumulative survival.

### Example 11. Vaccine Trial in Mice

The ability of a composition comprising a suspension of biodegradable particles, a co-polymer adjuvant, and an antigen to induce a protective T cell response in mice was tested under the parameters of Table 11.

**Table 11**

| | |
|---|---|
| Model System | Vaccine Trial in Mice |
| | *C. abortus* respiratory challenge model, termination day-11 post-challenge. Analyze body weight change and lung weight on day-11 post-challenge, body weight change (loss) from day 2 - day-11 post-challenge. |
| Mouse strain | 129S6, 6 weeks old at treatment |
| Challenge | 3×10⁸ *C. abortus* elementary bodies 6 week after treatment |
| Treatment | 1× subcutaneous in 200 µl suspension buffer |
| Carriers/ Controls | 1. Low peptide vaccines: 6.5 µg PLGA-PEG & 3.6 µg Pluronic L121® + 0.02 or 0.2 fM C. *abortus* peptides |
| | 2. High peptide vaccine: 6.5 µg PLGA-PEG & 3.6 µg Pluronic L121® + 2.0 fM C. *abortus* peptides |
| | 3. Vaccine carrier: 6.5 µg PLGA-PEG & 3.6 µg Pluronic L121® |
| | 4. Live vaccine: low-dose *C. abortus* intranasal inoculation (mediates maximum protection) |
| Antigen/ Vaccine Dose | 0.0, 0.02, 0.2, or 2.0 femtoMoles of each overlapping 20-mer peptide from the 5 best protective *C. abortus* proteins in a total of 10 µg vaccine composed of microparticles having an average effective diameter of ∼2 µm |
| Conclusion | Effective low-antigen dose vaccination is possible with a single 10 µg spray-dried microparticle vaccination using PLGA-PEG as a carrier and a co-polymer as adjuvant. Increasing the peptide antigen dose from 0.02 or 0.2 fM to 2.0 fM modulates the vaccine T cell immune response of mice from non-protective/pathogenic to protective. |

Vaccine compositions were prepared by combining microparticles of PLGA-PEG (6.5 µg), Pluronic L121® (3.5 µg) as a co-polymer adjuvant, peptides 0.0, 0.02, 0.2, or 2.0 femtoMoles of each overlapping 20-mer peptide from 5 *C. abortus* proteins shown to be protective against infection (see U.S. Published Application No. 2012/0009220, the contents of which are incorporated herein by reference in their entirety), in 200 µl suspension buffer.

The vaccine compositions and controls (0.0 femtoMoles peptides and live vaccine) were administered subcutaneously between the shoulders of 6 week old mice (strain 129S6). The mice were challenged at 6 weeks post-vaccination by administering intranasally 10⁸ *C. abortus* elementary bodies and the body weight loss an lung weight gain were assessed. The results presented in Figure 11A, B, C, D, E, F, and G illustrate that mice administered the vaccine containing 2.0 femtoMoles peptides exhibited the lowest body weight loss, lowest Chlamydia loads, and lowest lung weight gain similar to the live vaccine, suggesting that a dose of 2.0 femtoMoles peptides modulated the T cell response from non-protective/pathogenic to protective.

### Example 12. Vaccine Trial in Chickens

The ability of a composition comprising a suspension of biodegradable particles, a co-polymer adjuvant, and an antigen to induce a protective T cell response in chickens was tested under the parameters of Table 12.

**Table 12**

| | |
|---|---|
| Model System | Vaccine Trial in Chickens |
| | Infectious bursal disease virus (IBDV) respiratory challenge model, termination day-7 post-challenge. |
| | Analyze weight and inflammation of Bursa of Fabricius on day-7 post-challenge. |
| Chickens | Standard broiler chickens, treatment on day of hatching |
| Challenge | IBDV suspension intranasal on 3 weeks after treatment |
| Treatment | 1× subcutaneous in 200 µl suspension buffer |
| Vaccines/ Controls | 1. Low Peptide Vaccine: 175.5 µg PLGA-PEG & 94.5 µg Pluronic L121® + 0.54 fmoles each IBDV peptide |
| | 2. High Peptide Vaccines: 175.5 µg PLGA-PEG & 94.5 µg Pluronic L121® + 3.82 or 27.0 or 191.2 fmoles each IBDV peptide |
| | 3. Suspension buffer-treated chickens and IBDV challenge |
| | 4. Suspension buffer-treated chickens and no IBDV challenge (no disease) |
| Vaccine Dose | Overlapping 20-mer peptides from all IBDV virus proteins in a total of 270 µg microparticle vaccine, allometrically scaled 27-fold for 1,600 g target weight of chickens at 3 weeks as compared to 10 µg microparticle vaccine for 20 g mouse target weight: (1,600/20)^{3/4} = 26.7. |
| Conclusion | Increase of peptide antigen dose at a single -270 microgram total vaccine dose modulates the vaccine T cell immune response of chickens from non-protective/pathogenic to protective in a vaccine delivered as spray-dried PLGA-PEG microparticles that contain co-polymer adjuvant. |

As indicated in Table 12, microparticles prepared from a solution of PLGA-PEG and Pluronic L121® (175.5 µg: 94.5 µg) and IBDV peptides consisting of 0.54, 3.82, 27.0, or 191.2 femtoMoles of overlapping 20-mer peptides from all IBDV virus proteins. In order to prepare the vaccine compositions, the microparticles were added to 200 µl of suspension buffer.

The vaccine compositions and control were administered subcutaneously to standard broiler chickens on the day of hatching. The chickens were challenged three weeks post-administration by administering IBDV. The weight and inflammation of Bursa of Fabricius were analyzed on day-7 post-challenge. Inflammation was scored on a scale of 1-4, and the increase of bursa weight caused by inflammation was corrected by dividing bursa weight with the inflammation score. The results presented in Figure 12A, B, C, and D suggest that a doses of 3.82 - 191.2 femtomoles (versus a dose of 0.54 femtomoles) modulated the T cell response from non-protective/pathogenic to protective in immunized chickens challenged with IBDV.

### Example 13. Vaccine Trial in Mice

The ability of a composition comprising a suspension of biodegradable particles, a co-polymer adjuvant, and an antigen to induce a protective T cell response in mice was tested under the parameters of Table 13.

**Table 13**

| | |
|---|---|
| Model System | Vaccine Trial in Mice |
| | *C*. *abortus* respiratory challenge model, termination day-11 post-challenge. Analyze body weight change and lung weight on day-11 post-challenge. |
| Mouse strain | 129S6, 6 weeks old at treatment |
| Challenge | 3×10⁸ *C*. *abortus* elementary bodies 6 week after treatment |
| Treatment | 1x subcutaneous in 200 µl suspension buffer |
| Carriers/ Controls | 1. Vaccine: 6.5 µg PLA L206S & 3.6 µg Pluronic L121® + *C. abortus* peptides |
| | 2. Vaccine Carrier: 6.5 µg PLA L206S & 3.6 µg Pluronic L121® |
| | 3. Live Vaccine: low-dose *C. abortus* intranasal inoculation (mediates maximum protection) |
| Antigen/ Vaccine Dose | 2.0 femtoMoles of each overlapping 20-mer peptide from the 5 best protective *C. abortus* proteins in a total of 10 µg vaccine composed of ∼2 µm microparticles |
| Conclusion | Different polymers are affective as carriers for a vaccine delivered as spray-dried polymer microparticles that contain co-polymer adjuvant. This effect occurs at a single ∼10 microgram total dose of the vaccine. |

As indicated in Table 13, microparticles were prepared from PLGA L206S (6.5 µg) and added to Pluronic L121 (3.6 µg) together with 2.0 femtomoles of each overlapping 20-mer peptide from 5 protective *C. abortus* proteins (see U.S. Published Application No. 2012/0009220, the contents of which are incorporated herein by reference in their entirety) in a total of 10 µg to form a vaccine. *(See* Vaccine 1. In Table 13). Microparticles also were prepared from PLGA-PEG (6.5 µg) and added to Pluronic L121 (3.6 µg) to form a carrier as a control. (*See* Vaccine Carrier 2. In Table 13). A live vaccine was utilized as a control. (*See* Live Vaccine 3., Table 13). The vaccine compositions and controls were administered intranasally (20 µl) to 6 week old mice (strain 129S6). The mice were challenged at 6 weeks post-administration by administering intranasally 10⁸ *C. abortus* elementary bodies. The results presented in Figure 13A, B, C, and D illustrate that mice administered the vaccine containing the 2.0 femtomoles exhibited a low body weight loss, a low lung weight gain, a low *C. abortus* load, and a low percent body weight loss versus day after challenge, similar to the live vaccine.

It will be readily apparent to one skilled in the art that varying substitutions and modifications may be made to the invention disclosed herein without departing from the scope and spirit of the invention. The invention illustratively described herein suitably may be practiced in the absence of any element or elements, limitation or limitations which is not specifically disclosed herein. The terms and expressions which have been employed are used as terms of description and not of limitation, and there is no intention in the use of such terms and expressions of excluding any equivalents of the features shown and described or portions thereof, but it is recognized that various modifications are possible within the scope of the invention. Thus, it should be understood that although the present invention has been illustrated by specific embodiments and optional features, modification and/or variation of the concepts herein disclosed may be resorted to by those skilled in the art, and that such modifications and variations are considered to be within the scope of this invention.

Citations to a number of patent and non-patent references are made herein. The cited references are incorporated by reference herein in their entireties. In the event that there is an inconsistency between a definition of a term in the specification as compared to a definition of the term in a cited reference, the term should be interpreted based on the definition in the specification.

## Claims

1. A composition for use in a method for stimulating an innate immune response in a subject, the method comprising administering a composition comprising biodegradable particles, the biodegradable particles having an effective average diameter of 0.1-20 µm, wherein the composition does not comprise an antigen for inducing adaptive immunity and wherein the composition comprises a surfactant.

2. The composition for use of claim 1, wherein the biodegradable particles comprise carbohydrate monomers.

3. The composition for use of claim 1 or claim 2, wherein the biodegradable particles are administered to the subject at a dose level between (BW/20)^{3/4} µg particles/BW and 100 x ((BW/20)^{3/4}) µg particles/BW, wherein BW is the body weight of the subject in grams.

4. The composition for use of any one of claims 1 to 3, wherein the particles have an effective average diameter of 0.5-10 µm.

5. The composition for use of any one of claims 1 to 4, wherein the particles comprises polylactide (PLA), optionally wherein the polylactide comprises poly(lactic-co-glycolic acid) (PLGA).

6. The composition for use of any one of claims 1 to 5, wherein the surfactant is a non-ionic surfactant.

7. The composition for use of any one of claims 1 to 6, wherein the composition further comprises a powder excipient for the biodegradable particles.

8. The composition for use of any one of claims 1 to 7, wherein the biodegradable particles are formed by a process that includes spray-drying.

9. The composition for use of any one of claims 1 to 8, wherein the composition comprises a suspension of the biodegradable particles.

10. The composition for use of any one of claims 1 to 9, wherein the composition further comprises an adjuvant.

11. The composition for use of any one of claims 1 to 10, wherein the composition further comprises an apoptosis inhibitor.

12. The composition for use of any one of claims 1 to 11, wherein the subject is a fowl, swine or ruminant.

13. The composition for use of any one of claims 1 to 12, wherein the composition is administered via intradermal, intramuscular, intraperitoneal, subcutaneous, intranasal, intrapulmonary, oral, intravaginal or intrarectal routes.
